# EUROPEAN PATENT SPECIFICATION

(11) **EP 2 567 235 B1**
(45) Date of publication and mention of the grant of the patent: **30.08.2017**
(21) Application number: 11777047.9
(22) Date of filing: 04.05.2011
(51) Int. Cl.: G01N 33/574, C12Q 1/68

(54) **METHOD FOR THE DIAGNOSIS OF EPITHELIAL CANCERS BY THE DETECTION OF EPICD POLYPEPTIDE**
VERFAHREN ZUR DIAGNOSE VON EPITHELKARZINOMEN DURCH NACHWEIS EINES EPICD-POLYPEPTIDS
PROCÉDÉ POUR LE DIAGNOSTIC DE CANCERS ÉPITHÉLIAUX PAR DÉTECTION DE POLYPEPTIDE EPICD

(30) Priority: 07.05.2010 US 332358 P; 04.05.2010 US 330966 P
(43) Date of publication of application: 13.03.2013
(73) Proprietor: Walfish, Paul, Toronto, Ontario M5R 3V7 (CA); Ralhan, Ranju, Thornhill, Ontario L4J 6T2 (CA)
(72) Inventor: Walfish, Paul, Toronto, Ontario M5R 3V7 (CA); Ralhan, Ranju, Thornhill, Ontario L4J 6T2 (CA)
(74) Representative: Schiweck, Weinzierl & Koch Patentanwälte Partnerschaft mbB
(86) International application number: PCT/CA2011/000511
(87) International publication number: WO 2011/137513

(56) References cited:
- WO-A2-2004/093808
- WO-A2-2012/153186
- DOROTHEA MAETZEL ET AL: "Nuclear signalling by tumour-associated antigen EpCAM", NATURE CELL BIOLOGY, vol. 11, no. 2, 1 February 2009 (2009-02-01), pages 162-171, XP055011161, ISSN: 1465-7392, DOI: 10.1038/ncb1824
- MAETZEL ET AL.: 'Nuclear signalling by tumour-associated antigen EpCAM' NATURE CELL BIOLOGY vol. 11, no. 2, 2009, pages 162 - 171, XP055011161
- RALHAN ET AL.: 'EpCAM nuclear localization identifies aggressive thyroid cancer and is a marker for poor prognosis' BMC CANCER vol. 10, 25 June 2010, pages 331 - 341, XP021075153
- RALHAN ET AL.: 'Nuclear and cytoplasmic accumulation of Ep-ICD is frequently detected in human epithelial cancers' PLOS ONE vol. 5, no. 11, November 2010, pages E14130-1 - E14130-15, XP055068803
- STOECKLEIN ET AL.: 'Ep-CAM expression in squamous cell carcinoma of the esophagus: a potential therapeutic target and prognostic marker' BMC CANCER vol. 6, 2006, XP021016158

## Description

### FIELD OF THE INVENTION

The invention relates to compositions, kits, and methods for detecting, diagnosing, monitoring, and characterizing epithelial cancers.

### BACKGROUND OF THE INVENTION

Epithelial cell adhesion molecule (EpCAM) is a 40kDa transmembrane glycoprotein that serves important roles in cell adhesion, cell proliferation, differentiation, migration, cell cycle regulation and is implicated in cancer and stem cell signaling [Munz et al., 2009]. The molecular mechanisms that regulate EpCAM expression are not well understood. Recently, regulated intramembrane proteolysis (RIP) has been shown to act as its mitogenic signal transducer in vitro and in vivo [Maetzel et al., Nat Cell Biol. 2009;11:162-171]. The cleavage and shedding of EpCAM ectodomain, EpEx, by proteases-TACE and Presenilin-2, releases its intracellular domain (EpICD) that translocates to the nucleus. The association of EpICD with FHL2 and Wnt pathway components - β-catenin and Lef-1 forms a nuclear complex that binds DNA at Lef-1 consensus sites and induces gene transcription, leading to increased cell proliferation and has been shown to be oncogenic in immunodeficient mice [Maetzel 2009]. In view of the multiple roles of EpCAM as an oncogenic signal transducer, cell adhesion molecule and cancer stem cell marker [Litvinov et al., J Cell Biol. 1997;139:1337-1348; Munz et al., Cancer Res. 2009;69:5627-5629], it is important to establish the clinical significance of EpICD in human cancers.

Nuclear EpICD was recently reported in a preliminary study in human colon cancer, but not in the normal colonic epithelium [Maetzel, 2009]. In view of the tremendous heterogeneity in solid tumors, the clinical significance of nuclear EpICD in other human cancers remains to be established. In addition, WO 2004/093808 inter alia describes polypeptides, with some of them being capable of inducing or enhancing an immune response against EpCAM

### SUMMARY OF THE INVENTION

The present invention relates to the biomarkers EpICD polypeptides and
a polynucleotide encoding an EpICD polypeptide hereinafter collectively referred to as "Epithelial Cancer Markers"), and agents that interact with the biomarkers, for detecting, diagnosing, characterizing, and monitoring epithelial cancer, wherein the epithelial cancer is breast cancer, prostate cancer, lung cancer, pancreatic cancer, urinary bladder cancer, ovarian cancer, liver cancer, head and neck cancer or esophageal cancer (e.g., monitoring progression of the cancer or the effectiveness of a therapeutic treatment), identifying subjects with a predisposition to epithelial cancer, and determining patient survival. The Epithelial Cancer Markers can be used in characterizing the aggressiveness of an epithelial cancer. The Epithelial Cancer Markers can be used to determine metastatic potential or patient survival.

A method of the invention wherein Epithelial Cancer Marker(s) are assayed can have enhanced sensitivity and/or specificity relative to a method assaying other markers. The enhanced clinical sensitivity may be about a 5-10% increase, in particular 6-9% increase, more particularly 8% increase in sensitivity. In an embodiment, a method of the invention provides an epithelial cancer clinical sensitivity of at least about 80 to 99%, in particular 90 to 95%, more particularly 91%, 92%, 93%, or 94% epithelial cancer clinical sensitivity. In embodiments of the invention where EpICD is detected in a tumor sample the clinical sensitivity can be greater than about 80 to 90%, more particularly greater than about 80 to 85%, most particularly greater than about 83%, 84%, or 85%. Clinical sensitivity and specificity may be determined using methods known to persons skilled in the art.

In accordance with methods of the invention, an Epithelial Cancer Marker in a sample may be assessed by detecting the presence in the sample of (a) a polypeptide or polypeptide fragment corresponding to the marker; (b) a transcribed nucleic acid or fragment thereof having at least a portion with which the marker is substantially identical; and/or (c) a transcribed nucleic acid or fragment thereof, wherein the nucleic acid hybridizes with the marker.

Disclosed within the context of the invention is also a method for detecting Epithelial Cancer Markers associated with epithelial cancer in a patient comprising or consisting essentially of:
(a) obtaining a sample from a patient;
(b) detecting or identifying in the sample one or more Epithelial Cancer Markers and
(c) comparing the detected amount with an amount detected for a standard.

Disclosed within the context of the invention is a method for diagnosing an epithelial cancer in a subject, the method comprising:
(a) contacting a sample from a subject with a reagent capable of measuring a level of a target Epithelial Cancer Marker; and
(b) providing a diagnosis of an epithelial cancer in said subject based on an increase in the level of an Epithelial Cancer Marker in the sample from the subject over a control level obtained from similar samples taken from subjects who do not have the epithelial cancer or from the subject at a different time.

Disclosed within the context of the invention is a method for diagnosing an epithelial cancer in a patient comprising or consisting essentially of:
(a) detecting or identifying in the sample Epithelial Cancer Markers; and
(b) comparing the detected amount with an amount detected for a standard, wherein an increase in Epithelial Cancer Markers is diagnostic of the epithelial cancer.

The invention provides a method for diagnosing an epithelial cancer in a subject comprising:
(a) detecting a level of an EpICD polypeptide or a polynucleotide encoding an EpICD polypeptide in a sample from the subject; and
(b) comparing the level detected in the subject's sample to levels detected for a predetermined standard, wherein an increased level of EpICD polypeptide or polynucleotide encoding an EpICD polypeptide in the sample from the subject over that of the control level is
indicative of epithelial cancer, wherein the epithelial cancer is breast cancer, prostate cancer, lung cancer, pancreatic cancer, urinary bladder cancer, ovarian cancer, liver cancer, head and neck cancer or esophageal cancer.

In an aspect, the predetermined standard is a control level obtained from samples of the same type from subjects who do not have epithelial cancer; wherein an increased level of EpICD polypeptide or polynucleotide encoding an EpICD polypeptide in the sample from the subject over that of the control level is indicative of epithelial cancer.

The invention also provides a method for diagnosing an increased risk of an epithelial cancer, in a subject, the method comprising a) contacting a first sample from a subject at a first time with a diagnostic reagent that measures a first level of an EpICD polypeptide; and b) diagnosing an increased risk of an epithelial cancer, wherein the epithelial cancer is breast cancer, prostate cancer, lung cancer, pancreatic cancer, urinary bladder cancer, ovarian cancer, liver cancer, head and neck cancer or esophageal cancer in the subject based upon an increased level of EpICD polypeptide in the sample from the subject over that of (i) a first control level of EpICD polypeptide obtained from samples of the same type taken from subjects who do not have the epithelial cancer; or (ii) an earlier sample level of EpICD polypeptide obtained from samples of the same type taken from the same subject at an earlier time. In an aspect, a subject does not have an increased risk of developing an epithelial cancer if the first level is the same as either the first control level or the earlier sample level.

In a particular embodiment of the invention, a method is provided for diagnosing an epithelial cancer in a patient comprising or consisting essentially of:
(a) detecting or identifying in the sample Epithelial Cancer Markers and optionally EpEx (e.g. membranous EpEx), and
(b) comparing the detected amount with an amount detected for a standard, wherein an increase in Epithelial Cancer Markers and optionally a decrease or absence of EpEx is diagnostic of the epithelial cancer.

Also disclosed herein is a method for detecting Epithelial Cancer Markers in a patient comprising or consisting essentially of:
(a) obtaining a sample (e.g. tumor sample) from a patient;
(b) detecting in the sample Epithelial Cancer Markers; and
(c) comparing the detected amount with an amount detected for a standard or cut-off value.

The term "detect" or "detecting" includes assaying, or otherwise establishing the presence or absence of the target marker(s), subunits, or combinations of reagent bound targets, and the like, or assaying for ascertaining, establishing, characterizing, predicting or otherwise determining one or more factual characteristics of an epithelial cancer such as stage, aggressiveness, metastatic potential or patient survival, or assisting with same. A standard may correspond to levels quantitated for samples from control subjects with no disease or early stage disease (e.g., low grade epithelial cancer) or from other samples of the subject.

Disclosed within the context of the invention is a method of assessing whether a patient is at risk or afflicted with an epithelial cancer, the method comprising comparing:
(a) levels of Epithelial Cancer Markers from the patient; and
(b) standard levels of Epithelial Cancer Markers in samples of the same type obtained from control patients not afflicted with the epithelial cancer or with a lower grade of the epithelial cancer, wherein altered levels of Epithelial Cancer Markers relative to the corresponding standard levels of Epithelial Cancer Markers is an indication that the patient is at risk of or afflicted with the epithelial cancer.

In an aspect of a method of the invention for assessing whether a patient is at risk of or afflicted with an epithelial cancer, higher levels of Epithelial Cancer Markers, in a sample relative to corresponding normal levels or levels from a patient with a lower grade of epithelial cancer, is an indication that the patient is at risk of or afflicted with epithelial cancer.

In an embodiment of a method of the invention for assessing whether a patient is risk of or afflicted with epithelial cancer, levels of Epithelial Cancer Markers in a sample from the patient are compared to a standard, and higher levels of Epithelial Cancer Markers compared to a standard are indicative of epithelial cancer.

In an embodiment of a method of the invention for diagnosing breast cancer, levels of Epithelial Cancer Markers in a sample from the patient are compared to a standard.

In an embodiment of a method of the invention for diagnosing prostate cancer, levels of Epithelial Cancer Markers in a sample from the patient are compared to a standard.

In an embodiment of a method of the invention for diagnosing lung cancer, levels of Epithelial Cancer Markers in a sample from the patient are compared to a standard.

In an embodiment of a method of the invention for diagnosing pancreatic cancer, levels of Epithelial Cancer Markers in a sample from the patient are compared to a standard.

In an embodiment of a method of the invention for diagnosing urinary bladder cancer, levels of Epithelial Cancer Markers in a sample from the patient are compared to a standard.

In an embodiment of a method of the invention for diagnosing ovarian cancer, levels of Epithelial Cancer Markers in a sample from the patient are compared to a standard.

In an embodiment of a method of the invention for diagnosing liver cancer, levels of Epithelial Cancer Markers in a sample from the patient are compared to a standard.

In an embodiment of a method of the invention for diagnosing head and neck, levels of Epithelial Cancer Markers in a sample from the patient are compared to a standard.

In an embodiment of a method of the invention for diagnosing esophageal cancer, levels of Epithelial Cancer Markers in a sample from the patient are compared to a standard.

Disclosed within the context of the present invention are further methods used to diagnose the stage of an epithelial cancer in a subject or characterizing epithelial cancer in a subject. In an embodiment, the method comprises comparing
(a) levels of a Epithelial Cancer Marker from a sample from the patient; and
(b) levels of the Epithelial Cancer Marker in control samples of the same type obtained from patients without epithelial cancer or control patients with a different stage of epithelial cancer, wherein altered levels of the Epithelial Cancer Marker, relative to the corresponding levels in the control samples is an indication that the patient is afflicted with a more aggressive or metastatic epithelial cancer.

Disclosed within the context of the invention is further a non-invasive non-surgical method for detection or diagnosis of epithelial cancer in a subject comprising: obtaining a sample (e.g., biopsy sample) from the subject; subjecting the sample to a procedure to detect Epithelial Cancer Marker(s); detecting or diagnosing epithelial cancer by comparing the levels of Epithelial Cancer Marker(s) to the levels of Epithelial Cancer Marker(s) obtained from a control subject with no epithelial cancer or a lower grade of epithelial cancer.

In an aspect, the invention provides a method for monitoring the progression of an epithelial cancer in a patient the method comprising:
(a) detecting Epithelial Cancer Marker(s) in a patient sample (e.g. biopsy sample) at a first time point;
(b) repeating step (a) at a subsequent point in time; and
(c) comparing the levels detected in (a) and (b), and thereby monitoring the progression of the epithelial cancer wherein the epithelial cancer is breast cancer, prostate cancer, lung cancer, pancreatic cancer, urinary bladder cancer, ovarian cancer, liver cancer, head and neck cancer or esophageal cancer..

Disclosed within the context of the invention is a method for classifying a patient having epithelial cancer, the method comprising measuring Epithelial Cancer Marker(s) in a sample from the patient and correlating the values measured to values measured for the Epithelial Cancer Markers from epithelial cancer patients stratified in classification groups. The method can be used to predict patient survival, wherein the Epithelial Cancer Marker(s) are predictive of survival and wherein the classification groups comprise groups of known overall survival. In various embodiments the values measured can be normalized to provide more accurate quantification and to correct for experimental variations.

In particularly useful aspects of the invention, the Epithelial Cancer Markers detected are polynucleotides ("EpICD polynucleotides") and levels of EpICD polynucleotides in a sample (e.g., biopsy sample) from a patient are compared with EpICD polynucleotides levels from samples of patients without epithelial cancer, with a lower grade of epithelial cancer, or from levels from samples of the same patient. A method of the invention may employ one or more polynucleotides, oligonucleotides, or nucleic acids capable of hybridizing to EpICD polynucleotides. In an aspect of the invention, EpICD mRNA is detected.

Disclosed within the context of the present invention is a method for diagnosing and characterizing epithelial cancer, more particularly the stage of epithelial cancer, in a sample from a subject comprising isolating nucleic acids, preferably mRNA, from the sample, and detecting EpICD polynucleotides in the sample. In an embodiment, the presence of increased levels of EpICD polynucleotides in the sample compared to a standard or control is indicative of epithelial cancer.

Disclosed within the context of the invention are also methods for determining the presence or absence of an epithelial cancer or the aggressiveness or metastatic potential of an epithelial cancer in a subject comprising detecting in the sample a level of nucleic acids that hybridize to an EpICD polynucleotide, and comparing the level(s) with a predetermined standard or cut-off value, and therefrom determining the presence or absence of epithelial cancer or the aggressiveness or metastatic potential of an epithelial cancer in the subject. Disclosed herein is also a method for determining the aggressiveness or metastatic potential of epithelial cancer in a subject comprising (a) contacting a sample taken from the subject with oligonucleotides that hybridize to EpICD polynucleotides; and (b) detecting in the sample a level of nucleic acids that hybridize to the oligonucleotides relative to a predetermined standard or cut-off value, and therefrom determining the aggressiveness or metastatic potential of the cancer in the subject.

Disclosed within the context of the invention is also a method of assessing the aggressiveness or metastatic potential of an epithelial cancer in a patient, the method comprising comparing:
(a) levels of EpICD polynucleotides in a sample from the patient; and
(b) control levels of EpICD polynucleotides in samples of the same type obtained from control patients not afflicted with epithelial cancer or a lower grade of epithelial cancer, wherein altered levels of EpICD polynucleotides relative to the corresponding control levels of EpICD polynucleotides is an indication of the aggressiveness or metastatic potential of the epithelial cancer.

The method for assessing whether a patient is afflicted with an aggressive or metastatic epithelial cancer, higher levels of EpICD polynucleotides in a sample relative to the corresponding control levels is an indication that the patient is afflicted with an aggressive or metastatic epithelial cancer.

In an aspect, the invention provides a method for monitoring the progression of epithelial cancer in a patient, the method comprising:
(a) detecting EpICD polynucleotides in a patient sample at a first time point; and
(b) repeating step (a) at a subsequent point in time; and
(c) comparing the levels detected in (a) and (b), and thereby monitoring the progression of epithelial cancer in the patient.

Disclosed within the context of the invention is further a method of assessing the efficacy of a therapy for epithelial cancer in a patient. This method comprises comparing:
(a) levels of EpICD polynucleotides in a first sample obtained from the patient prior to providing at least a portion of the therapy to the patient; and
(b) levels of EpICD polynucleotides in a second sample obtained from the patient following therapy.

Significantly different levels of EpICD polynucleotides in the second sample, relative to the first sample, can be an indication that the therapy is efficacious for inhibiting epithelial cancer. In an embodiment, the method is used to assess the efficacy of a therapy for inhibiting epithelial cancer, more particularly aggressive or metastatic epithelial cancer, and lower levels of EpICD polynucleotides in the second sample relative to the first sample, is an indication that the therapy is efficacious for inhibiting the cancer or metastasis. The therapy may be any therapy for treating epithelial cancer including but not limited to chemotherapy, immunotherapy, gene therapy, radiation therapy, and surgical removal of tissue. Therefore, the method can be used to evaluate a patient before, during, and after therapy, for example, to evaluate the reduction in tumor burden, aggressiveness or metastatic potential of the tumor.

Within certain embodiments, the amount of nucleic acid that is mRNA is detected via amplification reactions such as polymerase chain reaction (PCR) using, for example, at least one oligonucleotide primer that hybridizes to an EpICD polynucleotide or a complement of such polynucleotide. Within other embodiments, the amount of mRNA is detected using a hybridization technique, employing an oligonucleotide probe that hybridizes to an EpICD polynucleotide, or a complement thereof.

When using mRNA detection, the method may be carried out by combining isolated mRNA with reagents to convert to cDNA according to standard methods; treating the converted cDNA with amplification reaction reagents along with an appropriate mixture of primers to produce amplification products; and analyzing the amplification products to detect the presence of EpICD polynucleotides in the sample. For mRNA the analyzing step may be accomplished using RT-PCR analysis to detect the presence of EpICD polynucleotides. The analysis step may be accomplished by quantitatively detecting the presence of EpICD polynucleotides in the amplification product, and comparing the quantity of EpICD polynucleotides, detected against a panel of expected values for known presence or absence in normal and malignant tissue (e.g., tissue from patients with a different stage of epithelial cancer), derived using similar primers.

Therefore, also disclosed within context of the invention is a method wherein mRNA is detected by (a) isolating mRNA from a sample and combining the mRNA with reagents to convert it to cDNA; (b) treating the converted cDNA with amplification reaction reagents and nucleic acid primers that hybridize to an EpICD polynucleotide to produce amplification products; (c) analyzing the amplification products to detect an amount of mRNA EpICD polynucleotide; and (d) comparing the amount of mRNA to an amount detected against a panel of expected values for normal tissue and malignant tissue (e.g., tissue from patients with a different stage of epithelial cancer) derived using similar nucleic acid primers.

Protein based methods can also be used for diagnosing and monitoring epithelial cancer, in particular the aggressiveness or metastatic potential of epithelial cancer in a subject comprising detecting EpICD polypeptides in a sample from the subject. EpICD polypeptides may be detected using a binding agent for EpICD polypeptides, preferably antibodies specifically reactive with EpICD polypeptides.

Disclosed within context of the invention is also a method of assessing whether a patient is afflicted with or at risk of epithelial cancer which comprises comparing:
(a) levels of EpICD polypeptides in a sample from the patient; and
(b) control levels of EpICD polypeptides in a non-cancer sample or sample from a patient with a lower grade of epithelial cancer, wherein significantly different levels of EpICD polypeptides in the sample from the patient compared with the control levels (e.g. higher in the patient samples) is an indication that the patient is afflicted with or at risk of epithelial cancer.

Disclosed within the context of the invention are methods for determining the presence or absence of epithelial cancer or the aggressiveness or metastatic potential of a epithelial cancer in a patient comprising the steps of (a) contacting a biological sample obtained from a patient with a binding agent that specifically binds to an EpICD polypeptide; and (b) detecting in the sample an amount of EpICD polypeptide that binds to the binding agent(s), relative to a predetermined standard or cut-off value, and therefrom determining the presence or absence of the epithelial cancer or the aggressiveness or metastatic potential of the epithelial cancer in the patient.

In an embodiment, a method for detecting, diagnosing, staging and monitoring epithelial cancer in a subject by quantitating an EpICD polypeptide in a biological sample from the subject comprises (a) reacting the biological sample with an antibody specific for the EpICD polypeptide which is directly or indirectly labeled with a detectable substance; and (b) detecting the detectable substance.

In another embodiment the invention provides for using antibodies to detect expression of EpICD polypeptides in a sample, the method comprising: (a) combining antibodies specific for EpICD polypeptides with a sample under conditions which allow the formation of antibody:protein complexes; and (b) detecting complex formation, wherein complex formation indicates expression of EpICD polypeptides in the sample. Expression may be compared with standards and is diagnostic of epithelial cancer or the aggressiveness or metastatic potential of the epithelial cancer.

In an aspect, the invention provides a method for monitoring the progression of epithelial cancer in a patient, the method comprising:
(a) detecting EpICD polypeptides in a patient sample at a first time point;
(b) repeating step (a) at a subsequent point in time; and
(c) comparing the levels detected in (a) and (b), and thereby monitoring the progression of epithelial cancer in the patient.

Disclosed within the context of the invention is further a method of assessing the efficacy of a therapy for epithelial cancer in a patient. This method comprises comparing:
(a) levels of EpICD polypeptides in a first sample obtained from the patient prior to providing at least a portion of the therapy to the patient; and
(b) levels of EpICD polypeptides in a second sample obtained from the patient following therapy.

Significantly different levels of EpICD polypeptides in the second sample, relative to the first sample, can be an indication that the therapy is efficacious for inhibiting epithelial cancer. In an embodiment, the method is used to assess the efficacy of a therapy for inhibiting epithelial cancer, more particularly aggressive or metastatic epithelial cancer, and lower levels of EpICD polypeptides in the second sample relative to the first sample, is an indication that the therapy is efficacious for inhibiting the cancer or metastasis. The therapy may be any therapy for treating epithelial cancer including but not limited to chemotherapy, immunotherapy, gene therapy, radiation therapy, and surgical removal of tissue. Therefore, the method can be used to evaluate a patient before, during, and after therapy, for example, to evaluate the reduction in tumor burden, aggressiveness or metastatic potential of the tumor.

Disclosed within the context of the invention is also a composition for diagnosing an epithelial cancer comprising Epithelial Cancer Markers or agents that interact with Epithelial Cancer Markers. In particular, a composition for diagnosing an epithelial cancer comprises EpICD polypeptides, or agents that bind to EpICD polypeptides, or hybridize to or amplify EpICD polynucleotides.

In an embodiment, the composition comprises a probe that specifically hybridizes to an EpICD polynucleotide or a fragment thereof, and a probe that specifically hybridizes to a EpICD polynucleotide or a fragment thereof. Also a composition is provided comprising a specific primer(s) pair capable of amplifying an EpICD polynucleotide using polymerase chain reaction methodologies. In a still further embodiment, the composition comprises a binding agent(s) (e.g. antibody) that binds to an EpICD polypeptide or a fragment thereof. Probes, primers, and binding agents can be labeled with a detectable substance.

In an embodiment, a diagnostic composition comprises antibodies specific for EpICD polypeptides. In an embodiment, a diagnostic composition comprises primers that amplify EpICD polynucleotides.

Also disclosed within the context of the present invention is a use of an agent that interacts with an Epithelial Cancer Marker in the manufacture of a composition for diagnosing epithelial cancer.

The methods of the invention may also comprise detecting additional markers associated with an epithelial cancer. In embodiments of the methods of the invention, EpCAM and membranous EpEx, and/or polynucleotides encoding same are detected.

Further, the amount of Epithelial Cancer Markers may be mathematically combined with other markers of epithelial cancer. In an embodiment the invention provides for detecting or diagnosing epithelial cancer in a subject comprising:
(a) determining the amount of Epithelial Cancer Markers in a sample from the subject;
(b) determining the amount of other markers associated with the epithelial cancer (e.g. EpCAM or EpEx);
(c) mathematically combining the results of step (a) and step (b) to provide a mathematical combination; and
(d) comparing or correlating the mathematical combination to the presence of epithelial cancer or aggressiveness or metastatic potential of epithelial cancer.

The combination is preferably compared to a mathematical combination for a predetermined standard.

The invention also includes kits for carrying out methods of the invention. In an aspect the invention provides a kit for detecting and/or diagnosing an epithelial cancer comprising agents that hybridize to or amplify polynucleotides encoding EPICD polypeptides. In a particular aspect, the invention provides a test kit for diagnosing or characterizing epithelial cancer in a subject which comprises an agent that interacts with an Epithelial Cancer Marker(s). In an embodiment, the kit comprises reagents for identifying and/or assessing levels of nuclear EpICD polypeptide.

Disclosed within the context of the present invention is an *in vivo* method comprising administering to a mammal one or more agent that carries a label for imaging and binds to an Epithelial Cancer Marker, and then imaging the mammal. According to a preferred aspect an *in vivo* method for imaging epithelial cancer is provided comprising:
(a) injecting a patient with an agent that binds to an Epithelial Cancer Marker(s), the agent carrying a label for imaging the epithelial cancer;
(b) allowing the agent to incubate *in vivo* and bind to the Epithelial Cancer Marker(s); and
(c) detecting the presence of the label localized to the epithelial cancer.

In an embodiment of the invention the agent is an antibody which recognizes the Epithelial Cancer Marker(s). In another embodiment of the invention the agent is a chemical entity which recognizes the Epithelial Cancer Marker(s).

The agent carries a label to image the Epithelial Cancer Marker(s). Examples of labels useful for imaging are radiolabels, fluorescent labels (e.g., fluorescein and rhodamine), nuclear magnetic resonance active labels, positron emitting isotopes detectable by a positron emission tomography ("PET") scanner, chemiluminescers such as luciferin, and enzymatic markers such as peroxidase or phosphatase. Short-range radiation emitters, such as isotopes detectable by short-range detector probes, can also be employed.

Also contemplated are the localization or imaging methods described herein using multiple markers for epithelial cancer.

In an aspect, the invention provides antagonists (e.g. antibodies) specific for an Epithelial Cancer Marker, in particular EpICD that can be used therapeutically to destroy or inhibit the growth of epithelial cancer cells, or to block activity. In addition, Epithelial Cancer Markers may be used in various immunotherapeutic methods to promote immune-mediated destruction or growth inhibition of tumors expressing Epithelial Cancer Markers.

Other objects, features and advantages of the present invention will become apparent from the following detailed description. It should be understood, however, that the detailed description and the specific examples while indicating preferred embodiments of the invention are given by way of illustration only, since various changes and modifications within the spirit and scope of the invention will become apparent to those skilled in the art from this detailed description.

### DESCRIPTION OF THE DRAWINGS

The invention will now be described in relation to the drawings in which:
Figure 1 shows immunohistochemical analysis of EpICD in breast cancer and prostate cancer. Arrows show EpICD nuclear staining. Original magnification x 40.
Figure 2 shows immunohistochemical analysis of EpICD in colon cancer, bladder cancer, ovarian cancer, lung cancer, liver cancer and pancreatic cancer. Original magnification x 40.
Figure 3 is a scatter plot showing EpICD membranous expression in different kinds of cancers.
Figure 4 is a scatter plot showing cytoplasmic EpICD expression in epithelial tissues and cancers.
Figure 5 is a scatter plot showing nuclear EpICD expression in epithelial tissues and cancers.
Figure 6 is an ROC curve of EpICD nuclear expression in breast cancer. AUC: 0.968. Sensitivity: 94.12. Specificity: 100. Criterion value: >1.7
Figure 7 is an ROC curve of EpICD nuclear expression in prostate cancer. AUC: 0.973. Sensitivity: 95.56. Specificity: 100. Criterion value: >2.67
Figure 8 shows EpICD and beta catenin immunostaining in head and neck cancer. Original magnification x 20.
Figure 9 shows EpICD immunostaining in head and neck cancer. Original magnification x 20.
Figure 10 (panels A, B and C) shows a Box Plot of (A) cytoplasmic EpICD staining in head and neck/oral cancer and (B) nuclear EpICD staining in head and neck/oral cancer; (C) an ROC curve of EpICD cytoplasmic and nuclear expression in neck/oral cancer.
Figure 11 shows immunohistochemical analysis of EpICD protein in esophageal tissues. Original magnification x 20.
Figure 12 shows immunofluorescence analysis of EpICD in breast cancer tissues. The magnification is shown by the scale bar.
Figure 13 shows immunofluorescence analysis of EpICD in colon cancer tissues. The magnification is shown by the scale bar.
Figure 14 shows immunofluorescence analysis of EpICD in prostate cancer tissues. The magnification is shown by the scale bar.

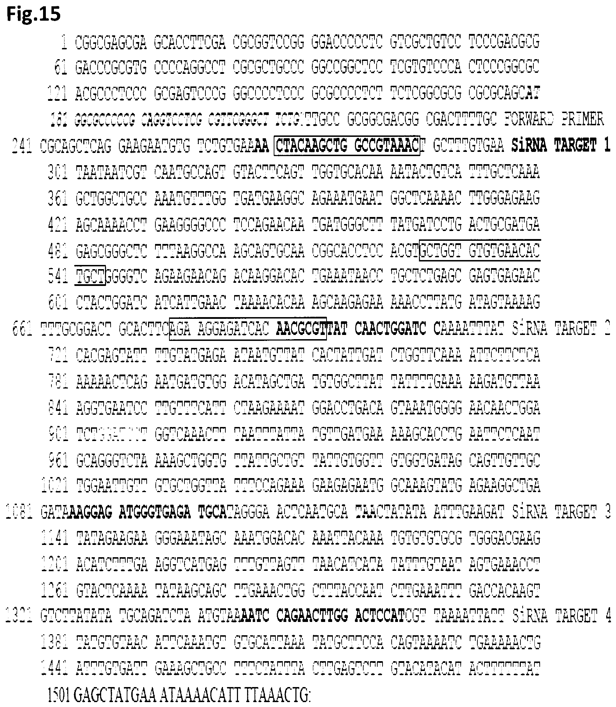

Figure 15 is a nucleic acid sequence encoding an EpICD Polypeptide showing siRNA targets [SEQ ID NOs. 3 and 5-10]

### DETAILED DESCRIPTION OF THE INVENTION

The invention relates to newly discovered correlations between expression of Epithelial Cancer Markers and epithelial cancers. The Epithelial Cancer Markers described herein provide methods for diagnosing, detecting or characterizing epithelial cancers. Methods are provided for diagnosing or detecting the presence or absence of an epithelial cancer in a sample, and for monitoring the progression of an epithelial cancer, as well as providing information about characteristics of an epithelial cancer that are relevant to the diagnosis and characterization of an epithelial cancer in a patient.

### Glossary

Unless defined otherwise, all technical and scientific terms used herein have the same meaning as commonly understood by one of ordinary skill in the art to which this invention belongs. The following definitions supplement those in the art and are directed to the present application and are not to be imputed to any related or unrelated case. Although any methods and materials similar or equivalent to those described herein can be used in the practice of the invention, particular materials and methods are described herein.

Numerical ranges recited herein by endpoints include all numbers and fractions subsumed within that range (e.g. 1 to 5 includes 1, 1.5, 2, 2.75, 3, 3.90, 4, and 5). It is also to be understood that all numbers and fractions thereof are presumed to be modified by the term "about." The term "about" means plus or minus 0.1 to 50%, 5-50%, or 10-40%, preferably 10-20%, more preferably 10% or 15%, of the number to which reference is being made. As used herein and in the appended claims, the singular forms "a", "an", and "the" include plural reference unless the context clearly dictates otherwise.

The term "epithelial cancer" refers to any malignant process that has an epithelial origin. Epithelial cancers detected in the present invention are are ovarian cancer, breast cancer, prostate cancer, lung cancer, pancreatic cancer, urinary bladder cancer, head and neck cancer, esophageal cancer and liver cancer. An epithelial cancer may be at different stages as well as varying degrees of grading. In embodiments, the epithelial cancer is selected from the group consisting of breast cancer, prostate cancer, lung cancer, pancreatic cancer, bladder cancer and ovarian cancer. In a particular embodiment, the epithelial cancer is breast cancer. In a particular embodiment, the epithelial cancer is ovarian cancer. In a particular embodiment, the epithelial cancer is prostate cancer. In a particular embodiment, the epithelial cancer is lung cancer. In a particular embodiment, the epithelial cancer is head and neck cancer. In a particular embodiment, the epithelial cancer is head and neck squamous cell carcinoma.

"Metastatic potential" refers to the ability or possibility of a cancer cell moving from the initial site to other sites in the body.

The term "sample" and the like mean a material known or suspected of expressing or containing Epithelial Cancer Markers, or binding agents such as antibodies specific for EpICD polypeptides. The sample may be derived from a biological source ("biological sample"), such as tissues, extracts, or cell cultures, including cells (e.g. tumor cells), cell lysates, and biological or physiological fluids, such as, for example, whole blood, plasma, serum, saliva, cerebral spinal fluid, sweat, urine, milk, peritoneal fluid and the like. A sample may be used directly as obtained from the source or following a pretreatment to modify the character of the sample, such as preparing plasma from blood, diluting viscous fluids, and the like. In certain aspects of the invention, the sample is a human physiological fluid, such as human serum. In certain aspects of the invention, the sample is a biopsy sample. In certain aspects of the invention the sample is a benign, malignant, or normal tissue sample.

The samples that may be analyzed in accordance with the invention include polynucleotides from clinically relevant sources, preferably expressed RNA or a nucleic acid derived therefrom (cDNA or amplified RNA derived from cDNA that incorporates an RNA polymerase promoter). As will be appreciated by those skilled in the art, the target polynucleotides can comprise RNA, including, without limitation total cellular RNA, poly(A)⁺ messenger RNA (mRNA) or fraction thereof, cytoplasmic mRNA, or RNA transcribed from cDNA (i.e., cRNA).

Target polynucleotides can be detectably labeled at one or more nucleotides using methods known in the art. The label is preferably uniformly incorporated along the length of the RNA, and more preferably, is carried out at a high degree of efficiency. The detectable label can be a luminescent label, fluorescent label, bio-luminescent label, chemi-luminescent label, radiolabel, and colorimetric label.

Target polynucleotides from a patient sample can be labeled differentially from polynucleotides of a standard. The standard can comprise target polynucleotides from normal individuals (e.g. those not afflicted with or pre-disposed to an epithelial cancer, in particular pooled from samples from normal individuals or patients with a different disease stage). The target polynucleotides can be derived from the same individual, but taken at different time points, and thus indicate the efficacy of a treatment by a change in expression of the markers, or lack thereof, during and after the course of treatment.

The terms "subject", "patient" and "individual" are used interchangeably herein and refer to a warm-blooded animal such as a mammal that is afflicted with, or suspected of having, at risk for or being pre-disposed to, or being screened for epithelial cancer, in particular actual or suspected epithelial cancer. The term includes but is not limited to domestic animals, sports animals, primates and humans. Preferably, the terms refer to a human.

A subject suspected of having epithelial cancer includes a subject that presents one or more symptoms indicative of an epithelial cancer (e.g., a noticeable lump or mass) or is being screened for a cancer (e.g., during a routine physical). A subject suspected of having an epithelial cancer may also have one or more risk factors. A subject suspected of having epithelial cancer has generally not been tested for cancer. However, a subject suspected of having epithelial cancer encompasses an individual who has received an initial diagnosis but for whom the stage of cancer is not known and people who once had cancer (e.g., an individual in remission).

A subject at risk for or being pre-disposed to epithelial cancer includes a subject with one or more risk factors for developing an epithelial cancer. Risk factors include, but are not limited to, gender, age, genetic predisposition, environmental exposure, previous incidents of cancer, pre-existing non-cancer diseases, and lifestyle.

As used herein, the term "characterizing epithelial cancer in a subject" refers to the identification of one or more properties of a cancer sample in a subject, including but not limited to the subject's prognosis or survival. Cancers may be characterized by the identification of the expression of one or more markers, including but not limited to, the Epithelial Cancer Markers disclosed herein.

"Polypeptide" and "protein" are used interchangeably herein and indicate at least one molecular chain of amino acids linked through covalent and/or non-covalent bonds. The terms include peptides, oligopeptides, and proteins, and post-translational modifications of the polypeptides, e.g. glycosylations, acetylations, phosphorylations, and the like. Protein fragments, analogues, mutated or variant proteins, fusion proteins, and the like, are also included within the meaning of the terms.

The term "EpCAM" refers to a type I membrane protein comprising an epidermal growth factor (EGF)-like domain and a thyroglobulin repeat domain. In particular, it is composed of a large extracellular domain (265 amino acids) (EpEx), a single transmembrane part of 23 amino acids (e.g. amino acids 266-288 in SEQ ID NO. 1), and a short cytoplasmic domain of 26 amino acids (e.g. EpICD, amino acids 289-314 in SEQ ID NO. 1). Two EGF-like repeats are located within the extracellular domain [Balzar et al., Mol Cell Biol. 2001 21(7):2570-80]. The mature enzyme consists of 314 amino acids. See Baeuerie PA and O Gires, British Journal of Cancer (2007) 96, pages 417-423 for a review of EpCAM (CD326).] The term includes native-sequence polypeptides, isoforms, polypeptide variants, precursors, and chimeric or fusion proteins of EpCAM, in particular human EpCAM. EpCAM polypeptides include, without limitation, polypeptides comprising the sequences found in Accession No. NP_002345 and SEQ ID NO. 1.

"Epithelial Cancer Markers" includes "EpICD polypeptides" and "EpICD polynucleotides".

The terms "EpICD polypeptides" and "EpICD" refer to a polypeptide consisting of the cytoplasmic domain of EpCAM. The cytoplasmic domain of EpCAM consists of 26 amino acids of EpCAM. In particular, the terms include the sequence consisting of amino acids 289-314 found in Accession No. NP_002345 and SEQ ID NO. 1. In embodiments of the invention, the EpICD polypeptide is a EpICD polypeptide associated with the nucleus.

A "native-sequence polypeptide" comprises a polypeptide having the same amino acid sequence of a polypeptide derived from nature. Such native-sequence polypeptides can be isolated from nature or can be produced by recombinant or synthetic means. The term specifically encompasses naturally occurring truncated or secreted forms of a polypeptide, polypeptide variants including naturally occurring variant forms (e.g. alternatively spliced forms or splice variants), and naturally occurring allelic variants.

The term "polypeptide variant" means a polypeptide having at least about 10%, 20%, 30%, 40%, 45%, 50%, 55%, 60%, 65%, 70%, 75%, 80%, 85%, 90%, 95%, 97%, 98%, or 99% amino acid sequence identity, particularly at least about 70-80%, more particularly at least about 85%, still more particularly at least about 90%, most particularly at least about 95%, 97%, or 99% amino acid sequence identity with a native-sequence polypeptide. Particular polypeptide variants have at least 70-80%, 85%, 90%, 95%, 97% or 99% amino acid sequence identity to sequences identified in Accession No. NP_002345 and SEQ ID NO: 1, in particular amino acids 289-314 found in Accession No. NP_002345 and SEQ ID NO: 1. Such variants include, for instance, polypeptides wherein one or more amino acid residues are added to, or deleted from, the N- or C-terminus of the full-length or mature sequences of the polypeptide, including variants from other species, but exclude a native-sequence polypeptide. In aspects of the invention variants retain the immunogenic activity of the corresponding native-sequence polypeptide.

Sequence identity of two amino acid sequences or of two nucleic acid sequences is defined as the percentage of amino acid residues or nucleotides in a candidate sequence that are identical with the amino acid residues in a polypeptide or nucleic acid sequence, after aligning the sequences and introducing gaps, if necessary, to achieve the maximum percent sequence identity, and not considering any conservative substitutions as part of the sequence identity. Alignment for purposes of determining percent amino acid or nucleic acid sequence identity can be achieved in various conventional ways, for instance, using publicly available computer software including the GCG program package (Devereux J. et al., Nucleic Acids Research 12(1): 387, 1984); BLASTP, BLASTN, and FASTA (Atschul, S.F. et al. J. Molec. Biol. 215: 403-410, 1990). The BLAST X program is publicly available from NCBI and other sources (BLAST Manual, Altschul, S. et al. NCBI NLM NIH Bethesda, Md. 20894; Altschul, S. et al. J. Mol. Biol. 215: 403-410, 1990). Skilled artisans can determine appropriate parameters for measuring alignment, including any algorithms needed to achieve maximal alignment over the full length of the sequences being compared. Methods to determine identity and similarity are codified in publicly available computer programs.

Polypeptide variants include polypeptides comprising amino acid sequences sufficiently identical to or derived from the amino acid sequence of a native polypeptide which includes fewer amino acids than the native polypeptides. A portion or fragment of a polypeptide can be a polypeptide which is for example, 3-5, 8-10, 10, 15, 15-20, 20, 25, 30, 35, 40, 45, 50,60, 70, 80, 90, 100 or more amino acids in length. Portions or fragments in which regions of a polypeptide are deleted can be prepared by recombinant techniques and can be evaluated for one or more functional activities such as the ability to form antibodies specific for a polypeptide. A portion or fragment of a polypeptide may comprise a domain of the polypeptide or a portion or fragment of such domain.

An allelic variant may also be created by introducing substitutions, additions, or deletions into a nucleic acid encoding a native polypeptide sequence or domain thereof such that one or more amino acid substitutions, additions, or deletions are introduced into the encoded protein. Mutations may be introduced by standard methods, such as site-directed mutagenesis and PCR-mediated mutagenesis. In an embodiment, conservative substitutions are made at one or more predicted non-essential amino acid residues. A "conservative amino acid substitution" is one in which an amino acid residue is replaced with an amino acid residue with a similar side chain, several of which are known in the art.

A naturally occurring allelic variant may contain conservative amino acid substitutions from the native polypeptide sequence or domain thereof, or it may contain a substitution of an amino acid from a corresponding position in polypeptide homolog, for example, a murine polypeptide.

A polypeptide disclosed herein includes chimeric or fusion proteins. A "chimeric protein" or "fusion protein" comprises all or part (preferably biologically active) of the polypeptide operably linked to a heterologous polypeptide (i.e., a different polypeptide). Within the fusion protein, the term "operably linked" is intended to indicate that the polypeptide and the heterologous polypeptide are fused in-frame to each other. The heterologous polypeptide can be fused to the N-terminus or C-terminus of the polypeptide. A useful fusion protein is a GST fusion protein in which a polypeptide is fused to the C-terminus of GST sequences. Another example of a fusion protein is an immunoglobulin fusion protein in which all or part of a polypeptide is fused to sequences derived from a member of the immunoglobulin protein family. Chimeric and fusion proteins can be produced by standard recombinant DNA techniques.

Polypeptides used in the methods disclosed herein may be isolated from a variety of sources, such as from human tissue types or from other sources, or prepared by recombinant or synthetic methods, or by any combination of these and similar techniques.

"Polynucleotide" refers to a polymeric form of nucleotides of any length, either ribonucleotides or deoxyribonucleotides. The term includes double- and single-stranded DNA and RNA, modifications such as methylation or capping and unmodified forms of the polynucleotide. The terms "polynucleotide" and "oligonucleotide" are used interchangeably herein. A polynucleotide may, but need not, include additional coding or non-coding sequences, or it may, but need not, be linked to other molecules and/or carrier or support materials. Polynucleotides for use in the methods of the invention may be of any length suitable for a particular method. In certain applications the term refers to antisense nucleic acid molecules (e.g. an mRNA or DNA strand in the reverse orientation to a sense EpICD polynucleotide).

"EpICD polynucleotides" include polynucleotides encoding an EpICD polypeptide. A polynucleotide encoding an EpCAM polypeptide that can be employed in the present invention includes, without limitation, nucleic acids comprising a sequence of Accession No. UniProtKB/TrEMBL Q6FG26 or SEQ ID NOs. 2 or 3 encoding an EpICD Polypeptide. A polynucleotide encoding EpICD that can be employed in the present invention includes, without limitation, nucleic acids comprising the sequence of Accession No. NM_002354_11 or SEQ ID NO. 4 encoding an EpICD Polypeptide.

Polynucleotides used in the methods of the invention include complementary nucleic acid sequences, and nucleic acids that are substantially identical to these sequences (e.g. at least about 10%, 20%, 30%, 40%, or 45%, preferably 50%, 55%, 60%, 65%, 70%, 75%, 80%, 85%, 90%, 95%, 97%, 98%, or 99% sequence identity).

Polynucleotides also include sequences that differ from a nucleic acid sequence due to degeneracy in the genetic code. As one example, DNA sequence polymorphisms within the nucleotide sequence of an Epithelial Cancer Marker disclosed herein may result in silent mutations that do not affect the amino acid sequence. Variations in one or more nucleotides may exist among individuals within a population due to natural allelic variation. DNA sequence polymorphisms may also occur which lead to changes in the amino acid sequence of a polypeptide.

Polynucleotides which may be used in the methods disclosed herein also include nucleic acids that hybridize under stringent conditions, preferably high stringency conditions to a nucleic acid sequence of an EpICD polynucleotide. Appropriate stringency conditions which promote DNA hybridization are known to those skilled in the art, or can be found in Ausubel et al., (eds) Current Protocols in Molecular Biology, John Wiley & Sons, N.Y. (1989), 6.3.1-6.3.6. Generally, stringent conditions may be selected that are about 5°C lower than the thermal melting point (Tm) for the specific sequence at a defined ionic strength and pH. The Tm is the temperature (under defined ionic strength, pH, and nucleic acid concentration) at which 50% of the probes complementary to a target sequence hybridize at equilibrium to the target sequence. Generally, stringent conditions will be those in which the salt concentration is less than about 1.0M sodium ion or other salts (e.g. about 0.01 to 1.0M sodium ion) and the temperature is at least about 30°C for short probes, primers or oligonucleotides (e.g. 10-50 nucleotides) and at least 60°C for longer probes, primers and oligonucleotides. For example, a hybridization may be conducted at 6.0 x sodium chloride/sodium citrate (SSC) at about 45°C, followed by a wash of 2.0 x SSC at 50°C, or at 42°C in a solution containing 6xSCC, 0.5% SDS and 50% formamide followed by washing in a solution of 0.1x SCC and 0.5% SDS at 68°C.

EpICD polynucleotides also include variant forms of the nucleic acids disclosed or referenced herein that arise by alternative splicing of an mRNA corresponding to a DNA. "Significantly different" levels of markers or a "significant difference" in marker levels in a patient sample compared to a control or standard (e.g. normal levels, levels from a different disease stage, or levels in other samples from a patient) may represent levels that are higher or lower than the standard error of the detection assay, preferably the levels are at least about 1.5, 2, 3, 4, 5, 6, 7, 8, 9 or 10 times higher or lower, respectively, than the control or standard.
"Microarray" and "array," refer to nucleic acid or nucleotide arrays or protein or peptide arrays that can be used to detect Epithelial Cancer Markers associated with epithelial cancer, for instance to measure gene or protein expression. A variety of arrays
are available commercially, such, for example, as the *in situ* synthesized oligonucleotide array GeneChip™ made by Affymetrix, Inc. or the spotted cDNA array, LifeArray™ made by Incyte Genomics Inc..

"Binding agent" refers to a substance such as a polypeptide, antibody, ribosome, or aptamer that specifically binds to an EpICD polypeptide. A substance "specifically binds" to an EpICD polypeptide if it reacts at a detectable level with the polypeptide, and does not react detectably with peptides containing unrelated sequences or sequences of different polypeptides. Binding properties may be assessed using an ELISA, which may be readily performed by those skilled in the art.

A binding agent may be a ribosome, with or without a peptide component, RNA or DNA molecule, or a polypeptide. A binding agent may be a polypeptide that comprises an EpICD polypeptide sequence, a peptide variant thereof, or a non-peptide mimetic of such a sequence. By way of example, an EpICD polypeptide sequence may be a peptide portion of the polypeptide that is capable of modulating a function mediated by the polypeptide.

An aptamer includes a DNA or RNA molecule that binds to nucleic acids and proteins. An aptamer that binds to an Epithelial Cancer Marker can be produced using conventional techniques, without undue experimentation. [For example, see the following publications describing *in vitro* selection of aptamers: Klug et al., Mol. Biol. Reports 20:97-107 (1994); Wallis et al., Chem. Biol. 2:543-552 (1995); Ellington, Curr. Biol. 4:427-429 (1994); Lato et al., Chem. Biol. 2:291-303 (1995); Conrad et al., Mol. Div. 1:69-78 (1995); and Uphoff et al., Curr. Opin. Struct. Biol. 6:281-287 (1996)].

Antibodies include, but are not limited to, synthetic antibodies, polyclonal antibodies, monoclonal antibodies, recombinantly produced antibodies, intrabodies, multispecific antibodies (including bi-specific antibodies), human antibodies, humanized antibodies, chimeric antibodies (for example, antibodies which contain the binding specificity of murine antibodies, but in which the remaining portions are of human origin), single-chain Fvs (scFv) (including bi-specific scFvs), single chain antibodies, immunologically active fragments (e.g. Fab fragments, F(ab') fragments, (Fab)₂ fragments), antibody light chains, disulfide-linked Fvs (sdFv), and anti-idiotypic (anti-Id) antibodies, and epitope-binding fragments of any of the above, or derivatives, such as enzyme conjugates or labelled derivatives. In particular, antibodies include immunoglobulin molecules and immunologically active portions of immunoglobulin molecules, i.e., molecules that contain an antigen binding site that immunospecifically bind to an EpICD Polypeptide.

Antibodies including monoclonal and polyclonal antibodies, fragments and chimeras, may be prepared using methods well known to those skilled in the art. Isolated native or recombinant EpICD polypeptides may be utilized to prepare antibodies. See, for example, Kohler et al. (1975) Nature 256:495-497; Kozbor et al. (1985) J. Immunol Methods 81:31-42; Cote et al. (1983) Proc Natl Acad Sci 80:2026-2030; and Cole et al. (1984) Mol Cell Biol 62:109-120 for the preparation of monoclonal antibodies; Huse et al. (1989) Science 246:1275-1281 for the preparation of monoclonal Fab fragments; and, Pound (1998) Immunochemical Protocols, Humana Press, Totowa, N.J for the preparation of phagemid or B-lymphocyte immunoglobulin libraries to identify antibodies. Antibodies specific for EpICD polypeptides may also be obtained from scientific or commercial sources. In an embodiment of the invention, antibodies are reactive against EpICD polypeptides if they bind with a Kₐ of greater than or equal to 10⁻⁷ M.

### General Methods

A variety of methods can be employed for the diagnostic and prognostic evaluation of an epithelial cancer and the identification of subjects with a predisposition to such conditions. Such methods may, for example, utilize EpICD polynucleotides and fragments thereof, and binding agents (e.g. antibodies) directed against EpICD polypeptides including peptide fragments. In particular, the polynucleotides and antibodies may be used, for example, for (1) the detection of the presence of polynucleotide mutations, or the detection of either over- or under-expression of mRNA, relative to a non-disorder state or the qualitative or quantitative detection of alternatively spliced forms of polynucleotide transcripts which may correlate with certain conditions or susceptibility toward such conditions; and (2) the detection of either an over- or an under-abundance of polypeptides relative to a non-disorder state or the presence of a modified (e.g., less than full length) polypeptide which correlates with a disorder state, or a progression toward a disorder state.

The methods described herein may be used to evaluate the probability of the presence of malignant cells, for example, in a group of cells freshly removed from a host. Such methods can be used to detect tumors, quantitate and monitor their growth, and help in the diagnosis and prognosis of disease. For example, higher levels of EpICD are indicative of an epithelial cancer or metastatic epithelial cancer, in particular breast cancer, head and neck cancer or prostate cancer.

Disclosed within the context of the invention is a method for determining the aggressiveness or stage of epithelial cancer comprising producing a profile of levels of EpICD polypeptides, and other markers associated with epithelial cancer, in cells from a patient, and comparing the profile with a reference to identify a profile for the test cells indicative of aggressiveness or stage of disease.

The methods of the invention require that the amount of Epithelial Cancer Markers quantitated in a sample from a subject being tested be compared to a predetermined standard or cut-off value. A standard may correspond to levels quantitated for another sample or an earlier sample from the subject, or levels quantitated for a control sample, in particular a sample from a subject with a lower grade cancer. Levels for control samples from healthy subjects or cancer subjects may be established by prospective and/or retrospective statistical studies. Healthy subjects who have no clinically evident disease or abnormalities may be selected for statistical studies. Diagnosis may be made by a finding of statistically different levels of Epithelial Cancer Markers compared to a control sample or previous levels quantitated for the same subject.

The invention also contemplates using multiple markers for epithelial cancer. Therefore, the invention contemplates a method for analyzing a biological sample for the presence of Epithelial Cancer Markers and other markers that are specific indicators of an epithelial cancer. The methods described herein may be modified by including reagents to detect the markers or polynucleotides encoding the markers. Other markers for breast cancer include, without limitation, BRCA1, BRCA2, urokinase plasminogen activator, plasminogen activator inhibitor, and CA27.29. Other markers for prostate cancer include, without limitation, prostate-specific antigen (PSA). Other markers for ovarian cancer include, without limitation, CA-125. The other markers may include EpCAM and EpEx.

### Nucleic Acid Methods

As noted herein an epithelial cancer may be detected based on the level of EpICD polynucleotides in a sample. Techniques for detecting nucleic acid molecules such as polymerase chain reaction (PCR) and hybridization assays are well known in the art.

Probes may be used in hybridization techniques to detect polynucleotides. The technique generally involves contacting and incubating nucleic acids obtained from a sample from a patient or other cellular source with a probe under conditions favorable for the specific annealing of the probes to complementary sequences in the nucleic acids (e.g. under stringent conditions as discussed herein). After incubation, the non-annealed nucleic acids are removed, and the presence of nucleic acids that have hybridized to the probe if any are detected.

Nucleotide probes for use in the detection of polynucleotide sequences in samples may be constructed using conventional methods known in the art. The probes may comprise DNA or DNA mimics corresponding to a portion of an organism's genome, or complementary RNA or RNA mimics. The nucleic acids can be modified at the base moiety, at the sugar moiety, or at the phosphate backbone. DNA can be obtained using standard methods such as polymerase chain reaction (PCR) amplification of genomic DNA or cloned sequences. Computer programs known in the art can be used to design primers with the required specificity and optimal amplification properties.

A nucleotide probe may be labeled with a detectable substance such as a radioactive label which provides for an adequate signal and has sufficient half-life such as ³²P, ³H, ¹⁴C or the like. Other detectable substances that may be used include antigens that are recognized by a specific labeled antibody, fluorescent compounds, enzymes, antibodies specific for a labeled antigen, and luminescent compounds. An appropriate label may be selected having regard to the rate of hybridization and binding of the probe to the nucleic acids to be detected and the amount of nucleic acids available for hybridization. Labeled probes may be hybridized to nucleic acids on solid supports such as nitrocellulose filters or nylon membranes as generally described in Sambrook et al., 1989, Molecular Cloning, A Laboratory Manual (2nd ed.). The nucleic acid probes may be used to detect EpICD polynucleotides, preferably in human cells. The nucleotide probes may also be useful in the diagnosis of epithelial cancer, involving EpICD polynucleotides in monitoring the progression of epithelial cancer, or monitoring a therapeutic treatment.

The detection of polynucleotides in a sample may involve the amplification of specific gene sequences using an amplification method such as PCR, followed by the analysis of the amplified molecules using techniques known to those skilled in the art. By way of example, oligonucleotide primers may be employed in a PCR based assay to amplify a portion of a polynucleotide and to amplify a portion of a polynucleotide derived from a sample, wherein the oligonucleotide primers are specific for (i.e. hybridize to) the polynucleotides. The amplified cDNA is then separated and detected using techniques well known in the art, such as gel electrophoresis.

In order to maximize hybridization under assay conditions, primers and probes employed in the methods of the invention generally have at least about 60%, preferably at least about 75% and more preferably at least about 90% identity to a portion of an EpICD polynucleotide; that is, they are at least 10 nucleotides, and preferably at least 20 nucleotides in length. In an embodiment the primers and probes are at least about 10-40 nucleotides in length.

Hybridization and amplification reactions may also be conducted under stringent conditions as discussed herein.

Hybridization and amplification techniques described herein may be used to assay qualitative and quantitative aspects of polynucleotide expression. For example, RNA may be isolated from a cell type or tissue known to express EpICD polynucleotides, and tested utilizing the hybridization (e.g. standard Northern analyses) or PCR techniques.

The primers and probes may be used *in situ* i.e., directly on tissue sections (fixed and/or frozen) of patient tissue obtained from biopsies or resections.

In an aspect the invention, employs reverse transcriptase-polymerase chain reaction (RT-PCR), in which PCR is applied in combination with reverse transcription. Generally, RNA is extracted from a sample tissue using standard techniques and is reverse transcribed to produce cDNA. The cDNA is used as a template for a polymerase chain reaction. The cDNA is hybridized to primer sets which are specifically designed against an EpICD polynucleotide. Once the primer and template have annealed a DNA polymerase is employed to extend from the primer, to synthesize a copy of the template. The DNA strands are denatured, and the procedure is repeated many times until sufficient DNA is generated to allow visualization by ethidium bromide staining and agarose gel electrophoresis.

Amplification may be performed on samples obtained from a subject with suspected epithelial cancer, an individual who is not afflicted with epithelial cancer or has early stage disease or has aggressive or metastatic disease. The reaction may be performed on several dilutions of cDNA spanning at least two orders of magnitude. A statistically significant difference in expression in several dilutions of the subject sample as compared to the same dilutions of the non-cancerous sample or early-stage cancer sample may be considered positive for the presence of cancer.

Oligonucleotides or longer fragments derived from EpICD polynucleotides may be used as targets in a microarray. The microarray can be used to monitor the expression levels of the polynucleotides and to identify genetic variants, mutations, and polymorphisms. The information from the microarray may be used to determine gene function, to understand the genetic basis of a disorder, to diagnose a disorder, and to develop and monitor the activities of therapeutic agents. Thus, the invention also includes an array comprising EpICD polynucleotides, and optionally other epithelial cancer markers. The array can be used to assay expression of EpICD polynucleotides in the array. The invention allows the quantitation of expression of the polynucleotides.

Provided are microarrays comprising EpICD polynucleotides. In one embodiment, a microarray for distinguishing samples associated with epithelial cancer comprising a positionally-addressable array of polynucleotide probes bound to a support, the polynucleotide probes comprising sequences complementary and hybridizable to EpICD polynucleotides is provided.

In an embodiment, the array can be used to monitor the time course of expression of EpICD polynucleotides in the array. This can occur in various biological contexts such as tumor progression. An array can also be useful for ascertaining differential expression patterns of EpICD polynucleotides, and optionally other epithelial cancer markers in normal and abnormal cells. This may provide a battery of nucleic acids that could serve as molecular targets for diagnosis or therapeutic intervention.

The preparation, use, and analysis of microarrays are well known to those skilled in the art. (See, for example, Brennan, T. M. et al. (1995) U.S. Pat. No. 5,474,796; Schena, et al. (1996) Proc. Natl. Acad. Sci. 93:10614-10619; Baldeschweiler et al. (1995), PCT Application WO95/251116; Shalon, D. et al. (I 995) PCT application WO95/35505; Heller, R. A. et al. (1997) Proc. Natl. Acad. Sci. 94:2150-2155; and Heller, M. J. et al. (1997) U.S. Pat. No. 5,605,662.)

### Protein Methods

Binding agents may be used for a variety of diagnostic and assay applications. There are a variety of assay formats known to the skilled artisan for using a binding agent to detect a target molecule in a sample. (For example, see Harlow and Lane, Antibodies: A Laboratory Manual, Cold Spring Harbor Laboratory, NY, 1988). In general, the presence or absence of an epithelial cancer in a subject may be determined by (a) contacting a sample from the subject with a binding agent; (b) detecting in the sample a level of polypeptide that binds to the binding agent; and (c) comparing the level of polypeptide with a predetermined standard or cut-off value. In particular aspects of the invention, the binding agent is an antibody.

The diagnostic method for monitoring or diagnosing epithelial cancer in a subject of the present invention can quantitate EpICD polypeptides in a biological sample from the subject comprising reacting the sample with antibodies specific for EpICD polypeptides which are directly or indirectly labeled with detectable substances and detecting the detectable substances.

The method for detecting or diagnosing an epithelial cancer of the present invention can comprise or consist essentially of:
(a) obtaining a sample suspected of containing EpICD polypeptides;
(b) contacting said sample with antibodies that specifically bind EpICD polypeptides under conditions effective to bind the antibodies and form complexes;
(c) measuring the amount of EpICD polypeptides present in the sample by quantitating the amount of the complexes; and
(d) comparing the amount of EpICD polypeptides present in the samples with the amount of EpICD polypeptides in a control, wherein a change or significant difference in the amount of EpICD polypeptides in the sample compared with the amount in the control is indicative of an epithelial cancer or risk of an epithelial cancer.

In an embodiment, the invention contemplates a method for monitoring the progression of epithelial cancer in an individual, comprising:
(a) contacting antibodies which bind to EpICD polypeptides with a sample from the individual so as to form complexes comprising the antibodies and EpICD polypeptides in the sample;
(b) determining or detecting the presence or amount of complex formation in the sample;
(c) repeating steps (a) and (b) at a point later in time; and
(d) comparing the result of step (b) with the result of step (c), wherein a difference in the amount of complex formation is indicative of disease, disease stage, progression, aggressiveness and/or metastatic potential of the cancer in said individual.

The amount of complexes may also be compared to a value representative of the amount of the complexes from an individual not at risk of, or afflicted with epithelial cancer at a different stage.

Antibodies specifically reactive with EpICD polypeptides or derivatives, such as enzyme conjugates or labeled derivatives, may be used to detect EpICD polypeptides in various samples (e.g. biological materials, in particular tissue samples). They may be used as diagnostic or prognostic reagents and they may be used to detect abnormalities in the level of EpICD polypeptides or abnormalities in the structure, and/or temporal, tissue, cellular, or subcellular location of EpICD polypeptides. Antibodies may also be used to screen potentially therapeutic compounds *in vitro* to determine their effects on epithelial cancer involving EpICD polypeptides. *In vitro* immunoassays may also be used to assess or monitor the efficacy of particular therapies.

Antibodies may be used in any immunoassay that relies on the binding interaction between antigenic determinants of EpICD polypeptides and the antibodies. Immunoassay procedures for *in vitro* detection of antigens in samples are also well known in the art. [See for example, Paterson et al., Int. J. Can. 37:659 (1986) and Burchell et al., Int. J. Can. 34:763 (1984) for a general description of immunoassay procedures]. Qualitative and/or quantitative determinations of EpICD polypeptides in a sample may be accomplished by competitive or non-competitive immunoassay procedures in either a direct or indirect format. Detection of EpICD polypeptides using antibodies can, for example involve immunoassays which are run in either the forward, reverse or simultaneous modes. Examples of immunoassays are radioimmunoassays (RIA), enzyme immunoassays (e.g. ELISA), immunofluorescence, immunoprecipitation, latex agglutination, hemagglutination, histochemical tests, and sandwich (immunometric) assays. Alternatively, the binding of antibodies to EpICD polypeptides can be detected directly using, for example, a surface plasmon resonance (SPR) procedure such as, for example, Biacore^{®}, microcalorimetry or nano-cantilivers. These terms are well understood by those skilled in the art, and they will know, or can readily discern, other immunoassay formats without undue experimentation.

Antibodies specific for EpICD polypeptides may be labelled with a detectable substance and localised in biological samples based upon the presence of the detectable substance. Examples of detectable substances include, but are not limited to, the hollowing: radioisotopes (e.g., ³H ¹⁴C, ³⁵S, ¹²⁵I, ¹³¹I), fluorescent labels, (e.g., FITC, rhodamine, lanthanide phosphors), luminescent labels such as luminol; and enzymatic labels (e.g., horseradish peroxidase, beta-galactosidase, luciferase, alkaline phosphatase, acetylcholinesterase), biotinyl groups (which can be detected by marked avidin e.g., streptavidin containing a fluorescent marker or enzymatic activity that can be detected by optical or calorimetric methods), and predetermined polypeptide epitopes recognized by a secondary reporter (e.g., leucine zipper pair sequences, binding sites for secondary antibodies, metal binding domains, epitope tags). In some embodiments, labels are attached via spacer arms of various lengths to reduce potential steric hindrance. Antibodies may also be coupled to electron dense substances, such as ferritin or colloidal gold, which are readily visualised by electron microscopy.

One of the ways an antibody can be detectably labelled is to link it directly to an enzyme. The enzyme when later exposed to its substrate will produce a product that can be detected. Examples of detectable substances that are enzymes are horseradish peroxidase, beta-galactosidase, luciferase, alkaline phosphatase, acetylcholinesterase, malate dehydrogenase, ribonuclease, urease, catalase, glucose-6-phosphate, staphylococcal nuclease, delta-5-steriod isomerase, yeast alcohol dehydrogenase, alpha-glycerophosphate, triose phosphate isomerase, asparaginase, glucose oxidase, and acetylcholine esterase.

For increased sensitivity in an immunoassay system a fluorescence-emitting metal atom such as Eu (europium) and other lanthanides can be used. These can be attached to the desired molecule by means of metal-chelating groups such as DTPA or EDTA.

A bioluminescent compound may also be used as a detectable substance. Examples of bioluminescent detectable substances are luciferin, luciferase and aequorin.

Indirect methods may also be employed in which the primary antigen-antibody reaction is amplified by the introduction of a second antibody, having specificity for the antibody reactive against an EpICD polypeptide. By way of example, if the antibody having specificity against an EpICD polypeptide is a rabbit IgG antibody, the second antibody may be goat anti-rabbit IgG, Fc fragment specific antibody labeled with a detectable substance as described herein.

Methods for conjugating or labelling the antibodies discussed above may be readily accomplished by one of ordinary skill in the art.

Cytochemical techniques known in the art for localizing antigens using light and electron microscopy may be used to detect EpICD polypeptides. Generally, an antibody may be labeled with a detectable substance and an EpICD polypeptide may be localized in tissues and cells based upon the presence of the detectable substance.

In the context of the methods of the invention, the sample, binding agents (e.g. antibodies), or EpICD polypeptides may be immobilized on a carrier or support, such as, for example, agarose, cellulose, nitrocellulose, dextran, Sephadex, Sepharose, liposomes, carboxymethyl cellulose, polyacrylamides, polystyrene, filter paper, ionexchange resin, plastic film, nylon or silk. The support material may have any possible configuration including spherical cylindrical or flat. Thus, the carrier may be in the shape of, for example, a tube, test plate, well, beads, disc, sphere, etc. The immobilized material may be prepared by reacting the material with a suitable insoluble carrier using known chemical or physical methods, for example, cyanogen bromide coupling. Binding agents (e.g. antibodies) may be indirectly immobilized using second binding agents specific for the first binding agent. For example, mouse antibodies specific for EpICD polypeptides may be immobilized using sheep anti-mouse IgG Fc fragment specific antibody coated on the carrier or support.

Where a radioactive label is used as a detectable substance, an EpICD polypeptide may be localized by radioautography. The results of radioautography may be quantitated by determining the density of particles in the radioautographs by various optical methods, or by counting the grains.

Time-resolved fluorometry may be used to detect a fluorescent signal, label, or detectable substance. For example, the method described in Christopoulos TK and Diamandis EP Anal. Chem., 1992:64:342-346 may be used with a conventional time-resolved fluorometer.

According to an embodiment of the invention, an immunoassay for detecting a EpICD polypeptide in a biological sample comprises contacting an amount of a binding agent that specifically binds to a EpICD polypeptide in the sample under conditions that allow the formation of complexes comprising the binding agent and EpICD polypeptide and determining the presence or amount of the complexes as a measure of the amount of the EpICD polypeptide contained in the sample.

In accordance with an embodiment of the invention, a method is provided wherein EpICD polypeptides antibodies are directly or indirectly labelled with enzymes, substrates for the enzymes are added wherein the substrates are selected so that the substrates, or a reaction product of an enzyme and substrate, form fluorescent complexes with lanthanide metals, preferably europium and terbium. A lanthanide metal(s) is added and EpICD polypeptides are quantitated in the sample by measuring fluorescence of the fluorescent complexes. Enzymes are selected based on the ability of a substrate of the enzyme, or a reaction product of the enzyme and substrate, to complex with lanthanide metals.

Examples of enzymes and substrates for enzymes that provide such fluorescent complexes are described in U.S. Patent No. 5,312,922 to Diamandis. By way of example, when the antibody is directly or indirectly labelled with alkaline phosphatase, the substrate employed in the method may be 4-methylumbelliferyl phosphate, 5-fluorosalicyl phosphate, or diflunisal phosphate. The fluorescence intensity of the complexes is typically measured using a time-resolved fluorometer.

Antibodies specific for EpICD polypeptides may also be indirectly labelled with enzymes. For example, an antibody may be conjugated to one partner of a ligand binding pair, and the enzyme may be coupled to the other partner of the ligand binding pair. Representative examples include avidin-biotin, and riboflavin-riboflavin binding protein.

Aspects of the methods of the invention involve (a) reacting a biological sample from a subject with antibodies specific for EpICD polypeptides wherein the antibodies are directly or indirectly labelled with enzymes; (b) adding substrates for the enzymes wherein the substrates are selected so that the substrates, or reaction products of the enzymes and substrates form fluorescent complexes; (c) quantitating EpICD polypeptides in the sample by measuring fluorescence of the fluorescent complexes; and (d) comparing the quantitated levels to levels obtained for other samples from the subject, or control subjects. In an embodiment, the EpICD polypeptide is nuclear EpICD and the quantitated level is compared to levels quantitated for normal subjects or subjects with an early stage of disease wherein an increase in the level of nuclear EpICD compared with the control subjects is indicative of an epithelial cancer and/or poor prognosis or survival.

A particular embodiment of the invention comprises the following steps:
(a) incubating a biological sample with a first antibody specific for EpICD polypeptides which is directly or indirectly labeled with a detectable substance, and a second antibody specific for EpICD polypeptides which is immobilized;
(b) separating the first antibody from the second antibody to provide a first antibody phase and a second antibody phase;
(c) detecting the detectable substance in the first or second antibody phase thereby quantitating EpICD polypeptides in the biological sample; and
(d) comparing the quantitated EpICD polypeptides with levels for a predetermined standard.

The standard may correspond to levels quantitated for samples from control subjects with no disease or early stage disease or from other samples of the subject. Increased levels of EpICD as compared to the standard may be indicative of an epithelial cancer or risk of an epithelial cancer.

In accordance with an embodiment, the present invention provides means for determining EpICD polypeptides in a sample by measuring EpICD polypeptides by immunoassay. It will be evident to a skilled artisan that a variety of competitive or non-competitive immunoassay methods can be used to measure EpICD polypeptides in samples, in particular fluid samples such as serum. Competitive methods typically employ immobilized or immobilizable antibodies to EpICD polypeptides and labeled forms of EpICD polypeptides. Sample EpICD polypeptides and labeled EpICD polypeptides compete for binding to antibodies specific for EpICD polypeptides. After separation of the resulting labeled EpICD polypeptides that have become bound to antibody (bound fraction) from that which has remained unbound (unbound fraction), the amount of the label in either bound or unbound fraction is measured and may be correlated with the amount of EpICD polypeptides in the test sample in any conventional manner, e.g., by comparison to a standard curve.

In another aspect, a non-competitive method is used for the determination of EpICD polypeptides with the most common method being the "sandwich" method. In this assay, two antibodies specific for an EpICD polypeptide are employed. One of the antibodies is directly or indirectly labeled (the "detection antibody"), and the other is immobilized or immobilizable (the "capture antibody"). The capture and detection antibodies can be contacted simultaneously or sequentially with the test sample. Sequential methods can be accomplished by incubating the capture antibody with the sample, and adding the detection antibody at a predetermined time thereafter or the detection antibody can be incubated with the sample first and then the capture antibody added. After the necessary incubation(s) have occurred, to complete the assay, the capture antibody may be separated from the liquid test mixture, and the label may be measured in at least a portion of the separated capture antibody phase or the remainder of the liquid test mixture. Generally it is measured in the capture antibody phase since it comprises EpICD polypeptide "sandwiched" between the capture and detection antibodies. In another embodiment, the label may be measured without separating the capture antibody and liquid test mixture.

In particular sandwich immunoassays of the invention mouse polyclonal/monoclonal antibodies specific for EpICD polypeptides and rabbit polyclonal/monoclonal antibodies specific for EpICD polypeptides are utilized.

In a typical two-site immunometric assay for EpICD polypeptides one or both of the capture and detection antibodies are polyclonal antibodies or one or both of the capture and detection antibodies are monoclonal antibodies (i.e. polyclonal/polyclonal, monoclonal/monoclonal, or monoclonal/polyclonal). The label used in the detection antibody can be selected from any of those known conventionally in the art. The label may be an enzyme or a chemiluminescent moiety, but it can also be a radioactive isotope, a fluorophor, a detectable ligand (e.g., detectable by a secondary binding by a labeled binding partner for the ligand), and the like. In an aspect, the antibody is labelled with an enzyme which is detected by adding a substrate that is selected so that a reaction product of the enzyme and substrate forms fluorescent complexes. The capture antibody may be selected so that it provides a means for being separated from the remainder of the test mixture. Accordingly, the capture antibody can be introduced to the assay in an already immobilized or insoluble form, or can be in an immobilizable form, that is, a form which enables immobilization to be accomplished subsequent to introduction of the capture antibody to the assay. An immobilized capture antibody may comprise an antibody covalently or noncovalently attached to a solid phase such as a magnetic particle, a latex particle, a microtiter plate well, a bead, a cuvette, or other reaction vessel. An example of an immobilizable capture antibody is antibody which has been chemically modified with a ligand moiety, e.g., a hapten, biotin, or the like, and which can be subsequently immobilized by contact with an immobilized form of a binding partner for the ligand, e.g., an antibody, avidin, or the like. In an embodiment, the capture antibody may be immobilized using a species specific antibody for the capture antibody that is bound to the solid phase.

### Screening Methods

Also disclosed within the context of the present invention are methods for evaluating test agents or compounds for their potential efficacy in treating an epithelial cancer. Test agents and compounds include but are not limited to peptides such as soluble peptides including Ig-tailed fusion peptides, members of random peptide libraries and combinatorial chemistry-derived molecular libraries made of D- and/or L-configuration amino acids, phosphopeptides (including members of random or partially degenerate, directed phosphopeptide libraries), antibodies [e.g. polyclonal, monoclonal, humanized, anti-idiotypic, chimeric, single chain antibodies, fragments, (e.g. Fab, F(ab)₂, and Fab expression library fragments, and epitope-binding fragments thereof)], polynucleotides (e.g. antisense, siRNA), and small organic or inorganic molecules. The agents or compounds may be endogenous physiological compounds or natural or synthetic compounds.

Also disclosed within the context of the present invention is a method for assessing the potential efficacy of a test agent in treating epithelial cancer comprising comparing:
(a) levels of one or more Epithelial Cancer Markers, and optionally other markers in a first sample obtained from a patient and exposed to the test agent; and
(b) levels of one or more Epithelial Cancer Markers, and optionally other markers, in a second sample obtained from the patient, wherein the sample is not exposed to the test agent, wherein a significant difference in the levels of expression of one or more Epithelial Cancer Markers, and optionally the other markers, in the first sample, relative to the second sample, is an indication that the test agent is potentially efficacious for treating epithelial cancer in the patient.

The first and second samples may be portions of a single sample obtained from a patient or portions of pooled samples obtained from a patient(s).

Disclosed within the context of the present invention is also a method of selecting an agent for treating epithelial cancer in a patient comprising:
(a) obtaining a sample from the patient;
(b) separately maintaining aliquots of the sample in the presence of a plurality of test agents;
(c) comparing one or more Epithelial Cancer Markers, and optionally other markers, in each of the aliquots; and
(d) selecting one of the test agents which alters the levels of one or more Epithelial Cancer Markers, and optionally other markers in the aliquot containing that test agent, relative to other test agents.

### Kits

The invention contemplates kits for carrying out the methods of the invention to detect an epithelial cancer. Such kits typically comprise two or more components required for performing a diagnostic assay. Components include but are not limited to compounds, reagents, containers, and/or equipment. Accordingly, the methods described herein may be performed by utilizing pre-packaged diagnostic kits comprising at least agents (e.g. antibodies, probes, primers, etc) described herein, which may be conveniently used, e.g., in clinical settings to diagnose patients afflicted with epithelial cancer, or exhibiting a predisposition to developing epithelial cancer and in particular to diagnose an epithelial cancer or risk of an epithelial cancer.

The invention contemplates a container with a kit comprising a binding agent(s) as described herein for determining an epithelial cancer. By way of example, the kit may contain antibodies specific for EpICD polypeptides, antibodies against the antibodies labelled with an enzyme(s), and a substrate for the enzyme(s). The kit may also contain microtiter plate wells, standards, assay diluent, wash buffer, adhesive plate covers, and/or instructions for carrying out a method of the invention using the kit.

Disclosed within the context of the present invention is a test kit for diagnosing an epithelial cancer in a subject which comprises an antibody that binds to EpICD polypeptides and/or polynucleotides that hybridize to or amplify EpICD polynucleotides. In another aspect the invention relates to use of a polynucleotide that hybridizes to or amplifies a EpICD polynucleotide.

The kit used in the invention can include antibodies or antibody fragments which bind specifically to epitopes of EpICD polypeptides and means for detecting binding of the antibodies to their epitopes associated with epithelial cancer cells, either as concentrates (including lyophilized compositions), which may be further diluted prior to testing. In particular, the invention provides a kit for diagnosing an epithelial cancer comprising a known amount of a first binding agent that specifically binds to EpICD polypeptides wherein the first binding agent comprises a detectable substance, or it binds directly or indirectly to a detectable substance.

A kit used in the invention may be designed to detect the levels of EpICD polynucleotides in a sample. Such kits generally comprise oligonucleotide probes or primers, as described herein, which hybridize to or amplify EpICD polynucleotides. Oligonucleotides may be used, for example, within PCR or hybridization procedures. Test kits useful for detecting target EpICD polynucleotides are also provided which comprise a container containing EpICD polynucleotide, and fragments or complements thereof. A kit can comprise one or more primers.

The kits used in the invention can further comprise containers with tools useful for collecting test samples (e.g. serum) including lancets and absorbent paper or cloth for collecting and stabilizing blood.

### Computer Systems

Analytic methods contemplated herein can be implemented by use of computer systems and methods described below and known in the art. Thus, the invention uses computer readable media comprising one or more Epithelial Cancer Markers. "Computer readable media" refers to any medium that can be read and accessed directly by a computer, including but not limited to magnetic storage media, such as floppy discs, hard disc storage medium, and magnetic tape; optical storage media such as CD-ROM; electrical storage media such as RAM and ROM; and hybrids of these categories such as magnetic/optical storage media. Thus, the invention contemplates the use of computer readable medium having recorded thereon markers identified for patients and controls.

"Recorded" refers to a process for storing information on computer readable medium. The skilled artisan can readily adopt any of the presently known methods for recording information on computer readable medium to generate manufactures comprising information on one or more markers disclosed herein.

A variety of data processor programs and formats can be used to store information on one or more Epithelial Cancer Markers. For example, the information can be represented in a word processing text file, formatted in commercially-available software such as WordPerfect and MicroSoft Word, or represented in the form of an ASCII file, stored in a database application, such as DB2, Sybase, Oracle, or the like. Any number of data processor structuring formats (e.g., text file or database) may be adapted in order to obtain computer readable medium having recorded thereon the marker information.

By providing the marker information in computer readable form, one can routinely access the information for a variety of purposes. For example, one skilled in the art can use the information in computer readable form to compare marker information obtained during or following therapy with the information stored within the data storage means.

A medium for holding instructions for performing a method for determining whether a patient has epithelial cancer, or a pre-disposition to such condition, comprising determining the presence or absence of one or more Epithelial Cancer Markers, and based on the presence or absence of the markers, determining the condition or a pre-disposition to the condition, optionally recommending a procedure or treatment is described within the context of the invention.

Disclosed within the context of the present invention is in an electronic system and/or in a network, a method for determining whether a subject has a condition disclosed herein, or a pre-disposition to a condition disclosed herein, comprising determining the presence or absence of one or more markers, and based on the presence or absence of the markers, determining whether the subject has the condition or a pre-disposition to the condition, and optionally recommending a procedure or treatment.

Further disclosed within the context of the present invention is in a network, a method for determining whether a subject has a condition disclosed herein or a pre-disposition to a condition disclosed herein comprising: (a) receiving phenotypic information on the subject and information on one or more markers disclosed herein associated with samples from the subject; (b) acquiring information from the network corresponding to the markers; and (c) based on the phenotypic information and information on the markers, determining whether the subject has the condition or a pre-disposition to the condition, and (d) optionally recommending a procedure or treatment.

Still further disclosed within the context of the present invention is a system for identifying selected records that identify a diseased cell or tissue. A system generally comprises a digital computer; a database server coupled to the computer; a database coupled to the database server having data stored therein, the data comprising records of data comprising one or more markers disclosed herein, and a code mechanism for applying queries based upon a desired selection criteria to the data file in the database to produce reports of records which match the desired selection criteria.

Disclosed within the context of the present invention is a business method for determining whether a subject has a condition disclosed herein or a pre-disposition to a condition disclosed herein comprising: (a) receiving phenotypic information on the subject and information on one or more markers disclosed herein associated with samples from the subject; (b) acquiring information from a network corresponding to the markers; and (c) based on the phenotypic information, information on the markers and acquired information, determining whether the subject has the condition or a pre-disposition to the condition, and optionally recommending a procedure or treatment.

Disclosed within the context of the present invention are computer systems, components, and methods described herein are used to monitor a condition (i.e. epithelial cancer) or determine the stage of a condition.

### Therapeutic Applications

Disclosed within the context of the present invention are therapeutic applications associated with the Epithelial Cancer Markers disclosed herein. Epithelial Cancer Markers may be a target for therapy. For example, EpICD can be a target for treatment of epithelial cancers. Therapeutic methods include immunotherapeutic methods including the use of antibody therapy. One or more antibodies can be used to treat or prevent epithelial cancer. Disclosed within the context of the present invention is also a method of preventing, inhibiting or reducing epithelial cancer comprising administering to a patient an antibody which binds to an EpICD polypeptide in an amount effective to prevent, inhibit, or reduce the condition or the onset of the condition.

An antibody which binds to an EpICD polypeptide may be in combination with a label, drug or cytotoxic agent, a target-binding region of a receptor, an adhesion molecule, a ligand, an enzyme, a cytokine, or a chemokine. In aspects of the invention, the antibody may be conjugated to cytotoxic agents (e.g., chemotherapeutic agents) or toxins or active fragments thereof. Examples of toxins and corresponding fragments thereof include diptheria A chain, exotoxin A chain, ricin A chain, abrin A chain, curcin, crotin, phenomycin, enomycin and the like. A cytotoxic agent may be a radiochemical prepared by conjugating radioisotopes to antibodies, or binding of a radionuclide to a chelating agent that has been covalently attached to the antibody. An antibody may also be conjugated to one or more small molecule toxins, such as a calicheamicin, a maytansine, a trichothene, and CC1065 (see U.S. Pat. No. 5,208,020).

Disclosed within the context of the present invention is also the administration of single antibodies as well as combinations, or "cocktails", of different individual antibodies such as those recognizing different epitopes of other markers. Such cocktails may have certain advantages inasmuch as they contain antibodies that bind to different epitopes of EpICD polypeptides and/or exploit different effector mechanisms. Such antibodies in combination may exhibit synergistic therapeutic effects. In addition, the administration of one or more marker specific antibodies may be combined with other therapeutic agents. The specific antibodies may be administered in their "naked" or unconjugated form, or may have therapeutic agents conjugated to them.

Disclosed within the context of the present invention is further a method of treating an epithelial cancer in a subject, comprising delivering to the subject in need thereof, an antibody specific for nuclear EpICD. In an aspect of the invention, the antibody is conjugated to a cytotoxic agent or toxin (see above). The antibody may be a therapeutic antibody disclosed for example in US Patent Nos. 7557190 and US7459538, US Published Application Nos. 20050163785 and 20070122406, and 20070196366 and McDonald et al.(Drug Design, Development and Therapy 2008;2:105-114). In a particular embodiment, the antibody is an antibody conjugated to a toxin, more particularly VB4-845 immunotoxin (Viventia Biotechnologies Inc., Ontario, Canada).

More particularly, there is provided a method of treating a subject having an epithelial cancer wherein an antibody specific for EpICD is administered in a therapeutically effective amount. In a further aspect, the antibody is provided in a pharmaceutically acceptable form.

Disclosed within the context of the present invention is a pharmaceutical composition for the treatment of an epithelial cancer characterized in that the composition comprises an antibody specific for EpICD together with a pharmaceutically acceptable carrier, excipient or vehicle.

Antibodies used in the methods of the invention may be formulated into pharmaceutical compositions comprising a carrier suitable for the desired delivery method. Suitable carriers include any material which when combined with the antibodies retains the function of the antibody and is non-reactive with the subject's immune systems. Examples include any of a number of standard pharmaceutical carriers such as sterile phosphate buffered saline solutions, bacteriostatic water, and the like (see, generally, Remington: The Science and Practise of Pharmacy 21st Edition. 2005, University of the Sciences in Philadelphia (Editor), Mack Publishing Company).

One or more marker specific antibody formulations may be administered via any route capable of delivering the antibodies to the site or injury. Routes of administration include, but are not limited to, intravenous, intraperitoneal, intramuscular, intradermal, and the like. Antibody preparations may be lyophilized and stored as a sterile powder, preferably under vacuum, and then reconstituted in bacteriostatic water containing, for example, benzyl alcohol preservative, or in sterile water prior to injection.

Treatment will generally involve the repeated administration of the antibody preparation via an acceptable route of administration at an effective dose. Dosages will depend upon various factors generally appreciated by those of skill in the art, including the etiology of the condition, stage of the condition, the binding affinity and half life of the antibodies used, the degree of marker expression in the patient, the desired steady-state antibody concentration level, frequency of treatment, and the influence of any therapeutic agents used in combination with a treatment method of the invention. A determining factor in defining the appropriate dose is the amount of a particular antibody necessary to be therapeutically effective in a particular context. Repeated administrations may be required to achieve a desired effect. Direct administration of one or more marker antibodies is also possible and may have advantages in certain situations.

Patients may be evaluated for Epithelial Cancer Markers in order to assist in the determination of the most effective dosing regimen and related factors. The assay methods described herein, or similar assays, may be used for quantitating marker levels in patients prior to treatment. Such assays may also be used for monitoring throughout therapy, and may be useful to gauge therapeutic success in combination with evaluating other parameters such as levels of markers.

EpICD polynucleotides disclosed herein can be turned off by transfecting a cell or tissue with vectors that express high levels of the polynucleotides. Such constructs can inundate cells with untranslatable sense or antisense sequences. Even in the absence of integration into the DNA, such vectors may continue to transcribe RNA molecules until all copies are disabled by endogenous nucleases. Vectors derived from retroviruses, adenovirus, herpes or vaccinia viruses, or from various bacterial plasmids, may be used to deliver polynucleotides to a targeted organ, tissue, or cell population. Methods well known to those skilled in the art may be used to construct recombinant vectors that will express polynucleotides such as antisense. (See, for example, the techniques described in Sambrook et al (supra) and Ausubel et al (supra).)

Methods for introducing vectors into cells or tissues include those methods discussed herein and which are suitable for *in vivo, in vitro* and *ex vivo* therapy. For example, delivery by transfection or by liposome are well known in the art.

Modifications of gene expression can be obtained by designing antisense molecules, DNA, RNA or PNA, to the regulatory regions of a polynucleotide, i.e., the promoters, enhancers, and introns. Preferably, oligonucleotides are derived from the transcription initiation site, e.g. between -10 and +10 regions of the leader sequence. The antisense molecules may also be designed so that they block translation of mRNA by preventing the transcript from binding to ribosomes. Inhibition may also be achieved using "triple helix" base-pairing methodology. Triple helix pairing compromises the ability of the double helix to open sufficiently for the binding of polymerases, transcription factors, or regulatory molecules. Therapeutic advances using triplex DNA are reviewed by Gee J E et al (In: Huber B E and B I Carr (1994) Molecular and Immunologic Approaches, Futura Publishing Co, Mt Kisco N.Y.).

Ribozymes are enzymatic RNA molecules that catalyze the specific cleavage of RNA. Ribozymes act by sequence-specific hybridization of the ribozyme molecule to complementary target RNA, followed by endonucleolytic cleavage. The invention therefore contemplates engineered hammerhead motif ribozyme molecules that can specifically and efficiently catalyze endonucleolytic cleavage of a polynucleotide marker.

Specific ribozyme cleavage sites within any potential RNA target may initially be identified by scanning the target molecule for ribozyme cleavage sites which include the following sequences, GUA, GUU and GUC. Once the sites are identified, short RNA sequences of between 15 and 20 ribonucleotides corresponding to the region of the target gene containing the cleavage site may be evaluated for secondary structural features which may render the oligonucleotide inoperable. The suitability of candidate targets may also be determined by testing accessibility to hybridization with complementary oligonucleotides using ribonuclease protection assays.

Also disclosed within the context of the present invention is a method of preventing, inhibiting, or reducing epithelial cancer in a patient comprising:
(a) obtaining a tumor sample from the patient;
(b) separately maintaining aliquots of the sample in the presence of a plurality of test agents;
(c) comparing levels of Epithelial Cancer Markers, and optionally one or more other markers of the epithelial cancer, in each aliquot;
(d) administering to the patient at least one test agent which alters the levels of Epithelial Cancer Markers, and optionally other markers of the epithelial cancer, in the aliquot containing that test agent, relative to the other test agents.

An active therapeutic substance described herein may be administered in a convenient manner by any standard route of administration, including without limitation, by injection (subcutaneous, intravenous, etc.), oral administration, inhalation, transdermal application, or rectal administration. Depending on the route of administration, the active substance may be coated in a material to protect the substance from the action of enzymes, acids and other natural conditions that may inactivate the substance. Solutions of an active substance as a free base or pharmaceutically acceptable salt can be prepared in an appropriate solvent with a suitable surfactant. Dispersions may be prepared in glycerol, liquid polyethylene glycols, and mixtures thereof, or in oils.

A composition described herein can be prepared by per se known methods for the preparation of pharmaceutically acceptable compositions which can be administered to subjects, such that an effective quantity of the active substance is combined in a mixture with a pharmaceutically acceptable vehicle. Suitable vehicles are described, for example, in Remington: The Science and Practice of Pharmacy (21st Edition. 2005, University of the Sciences in Philadelphia (Editor), Mack Publishing Company), and in The United States Pharmacopeia: The National Formulary (USP 24 NF19) published in 1999. On this basis, the compositions include, albeit not exclusively, solutions of the active substances in association with one or more pharmaceutically acceptable vehicles or diluents, and contained in buffered solutions with a suitable pH and iso-osmotic with the physiological fluids.

A composition is indicated as a therapeutic agent either alone or in conjunction with other therapeutic agents or other forms of treatment. The compositions may be administered concurrently, separately, or sequentially with other therapeutic agents or therapies.

The therapeutic activity of compositions and agents/compounds identified using a method disclosed within the context of the invention and may be evaluated *in vivo* using a suitable animal model.

The following non-limiting example is illustrative of the present invention:

### Example

### Immunohistochemical Analysis of Epithelial Cancer Markers

The following materials and methods were employed in the study described in this example:

### Antibodies

Anti-human-EpCAM mouse monoclonal antibody MOC-31(AbD Serotec, Oxford, UK, Raleigh, NC) recognizes an extracellular component (EpEx - EGF1 domain- aa 27-59) in the amino-terminal region of EpCAM [Myklebust et al, Cancer Res. 1993 Aug 15;53(16):3784-8.]. Anti-human rabbit monoclonal antibody, α- EpICD antibody 1144 (Epitomics, Burlingame, CA) recognizes the cytoplasmic domain of human EpCAM. β- catenin antibody was raised against aa 571-781 of β-catenin (Cat.# 610154, B D Sciences, San Jose, CA).

### Immunohistochemistry for EpEx and Ep-ICD expression in epithelial cancers

Serial epithelial cancer tissue sections (4µm thickness) were deparaffinized, hydrated in xylene and graded alcohol series. The slides were treated with 0.3% H₂O₂ at room temperature for 30 minutes to block the endogenous peroxidase activity. After blocking the non-specific binding with normal horse or goat serum, the sections were incubated with anti human antibodies -EpEx mouse monoclonal antibody MOC-31 (dilution 1:200), or α- EpICD rabbit monoclonal antibody 1144 (dilution 1:200), or mouse monoclonal β-catenin antibody (dilution 1:200) for 30 minutes and biotinylated secondary antibody (horse anti-mouse or goat antirabbit) for 30 minutes. The sections were finally incubated with VECTASTAIN Elite ABC Reagent (Vector labs, Burlingame, CA) and diaminobenzedine was used as the chromogen.

### Evaluation of immunohistochemical staining

Immunopositive staining was evaluated in five areas of the tissue sections as described [Ralhan et al., 2008, J Proteome Res. 2009 Jan;8(1):300-9]. Sections were scored as positive if epithelial cells showed immunopositivity in the plasma membrane, cytoplasm, and/or nucleus when observed by two evaluators who were blinded to the clinical outcome. These sections were scored as follows: 0, < 10% cells; 1, 10-30% cells; 2, 30-50% cells; 3, 50-70% cells; and 4, >70% cells showed immunoreactivity. Sections were also scored semi-quantitatively on the basis of intensity as follows: 0, none; 1, mild; 2, moderate; and 3, intense. Finally, a total score (ranging from 0 to 7) was obtained by adding the scores of percentage positivity and intensity for each of the epithelial cancer and normal epithelial tissue sections. The immunohistochemical data were subjected to statistical analysis.

### Statistical analysis

The immunohistochemical data were subjected to statistical analysis using SPSS 10.0 software (Chicago). Box plots were used to determine the distribution of total score of membranous EpEx, nuclear Ep-ICD and nuclear or cytoplasmic β-catenin expression in normal tissues and tumors. A cut-off = or > 2 was defined as positive criterion for nuclear β-catenin immunopositivity for statistical examination. For membranous β-catenin, score of 6 was defined as loss of expression. The correlation between expression of EpEx, Ep-ICD and/or β-catenin staining with overall patient survival was evaluated using life tables constructed from survival data with Kaplan- Meier plots.

### Immunofluorescence analysis

The immunofluorescence analysis of Ep-ICD and EpEx localization in human breast, colon and prostate carcinomas was performed fluorescent secondary antibodies. Human breast carcinoma, colon carcinoma and prostate carcinoma paraffin sections were incubated with either α-Ep-ICD rabbit monoclonal antibody 1144 (dilution 1:100) or mouse monoclonal antibody MOC-31 (dilution 1:100). For Ep-ICD, the secondary antibody used was a tetramethyl rhodamine isothiocyanate (TRITC)-labeled goat anti-rabbit antibody (Sigma-Aldrich, dilution 1:200). For EpEx, the secondary antibody used was a fluorescein isothiocyanate (FITC)-labeled goat anti-mouse antibody (Sigma-Aldrich, St. Louis, MO, 1:200 dilution). Nuclei were counterstained with DAPI (blue). Localization of EpEx (green) and EpICD (red) were assessed with Olympus Upright Flourescence Microscope (BX61) and images were captured by using Volocity software (PerkinElmer Waltham, MA)

### Results:

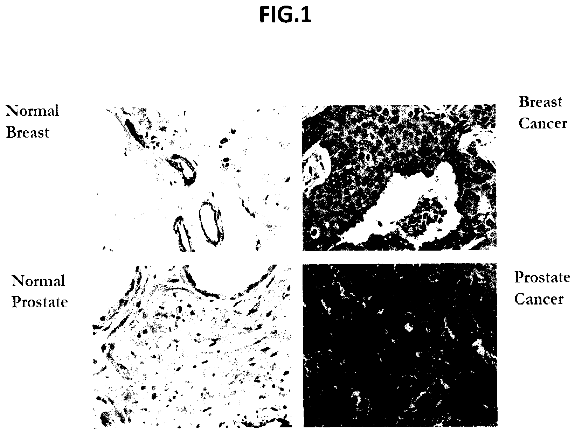

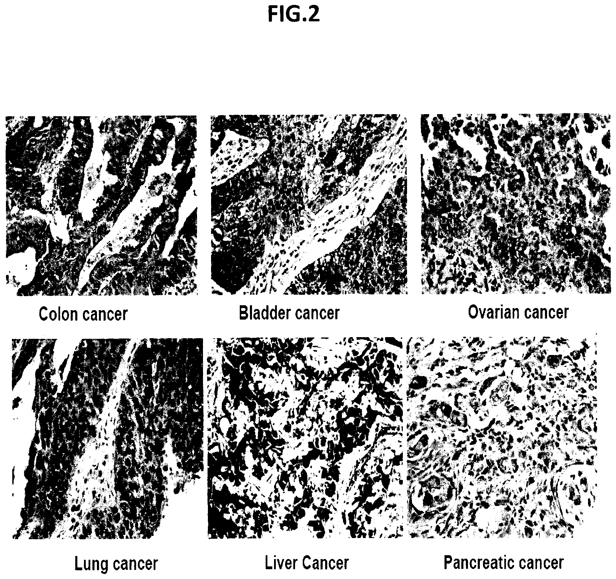

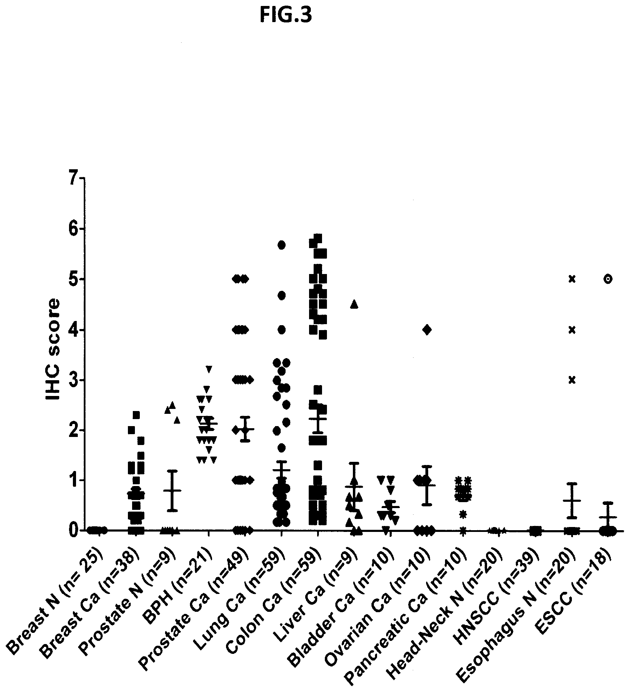

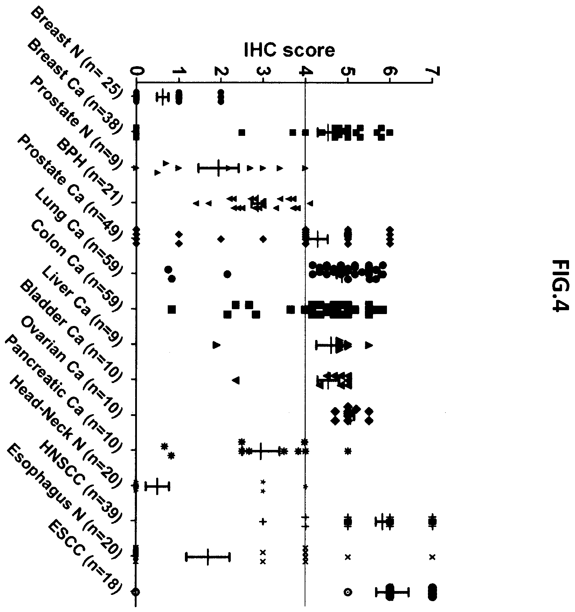

Immunohistochemical staining of paraffin tissue sections from a range of patients was conducted with a full-length monoclonal antibody to EpCAM (Ep-Ex) and a monoclonal antibody to the intracellular domain of EpCAM (Ep-ICD). Scoring analyses was performed by two independent observers for degrees of subcellular localization to the cell membrane, cytoplasm or nucleus. Nuclear EpICD was detected in tissues from patients with breast, prostate, colon and rectum, lung, pancreatic, urinary bladder, ovarian, liver and head and neck cancers. The results are shown in Figures 1 to 11 and the following Tables 1-6. Figure 1 shows nuclear Ep-ICD expression in representative breast and prostate tissue sections. Nuclear EpICD was also observed in tissues from patients with cancers of colon and rectum, urinary bladder, ovarian, lung, liver and pancreas (Figure 2), suggesting that nuclear Ep-ICD expression can be detected in several epithelial cancers. The distribution of Ep-ICD in plasma membrane of epithelial cells in normal and malignant breast tissues, normal and malignant prostate tissues, and in cancers of lung, colon and rectum, liver, urinary bladder, ovary and pancreas analysed are shown in the bar diagram in Figure 3. The cytoplasmic distribution of Ep-ICD in epithelial cells in normal and malignant breast tissues, normal and malignant prostate tissues, and in cancers of lung, colon and rectum, liver, urinary bladder, ovary and pancreas analysed are shown in the bar diagram in Figure 4.

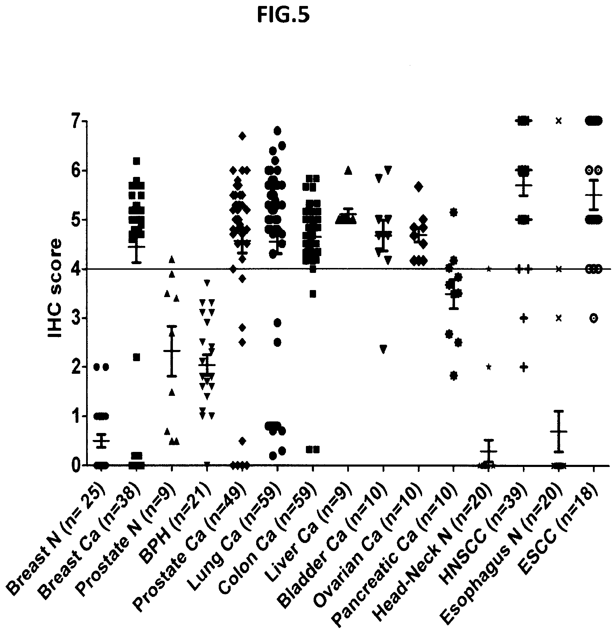

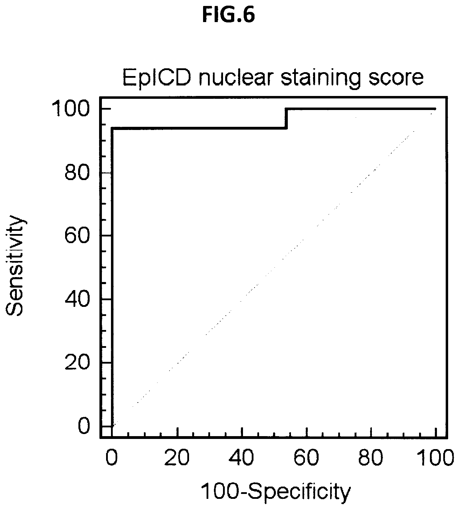

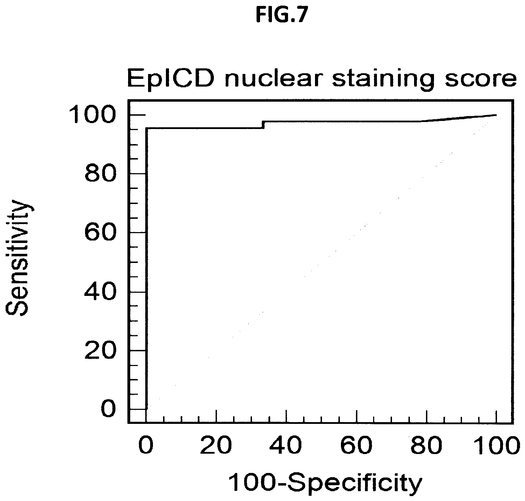

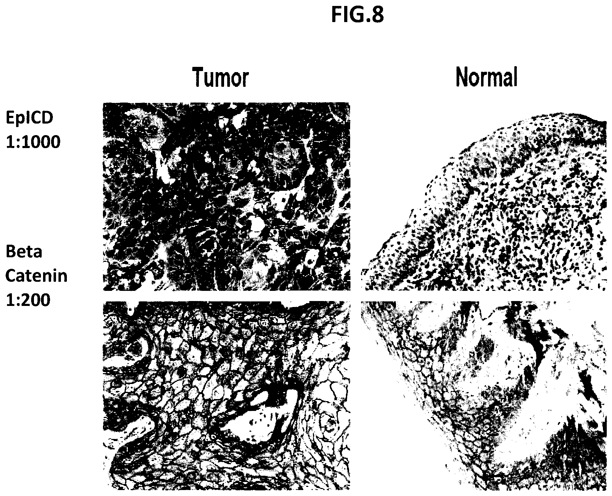

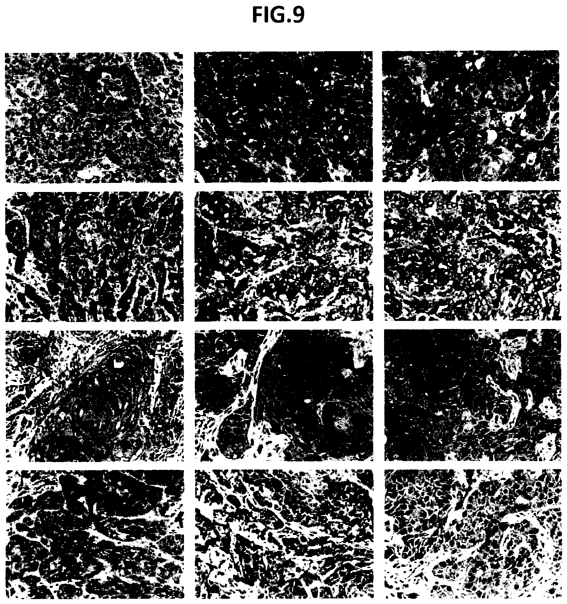

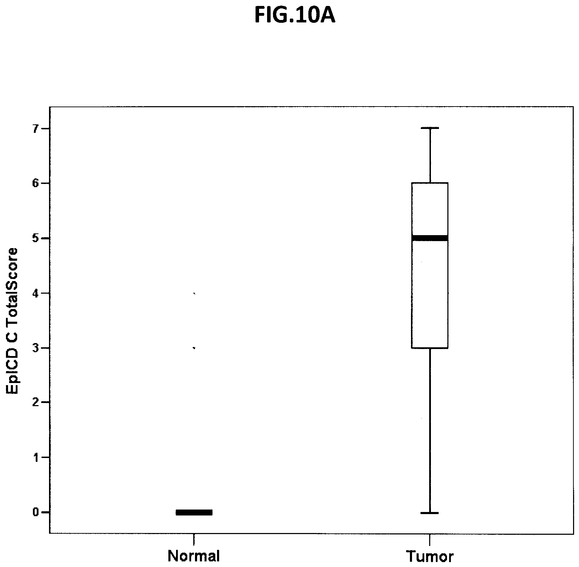

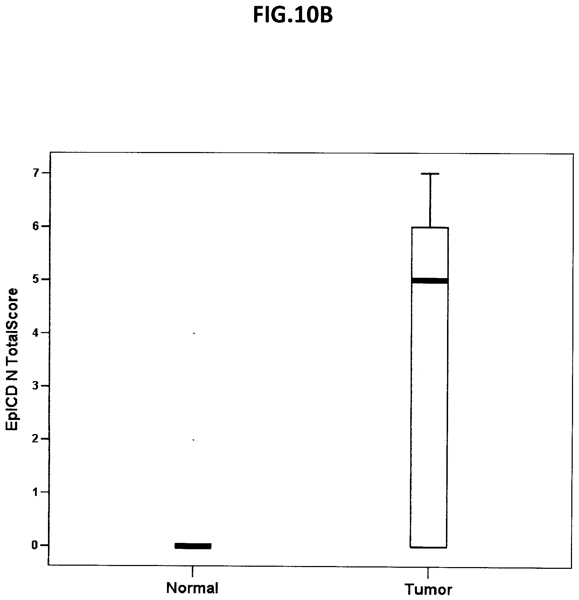

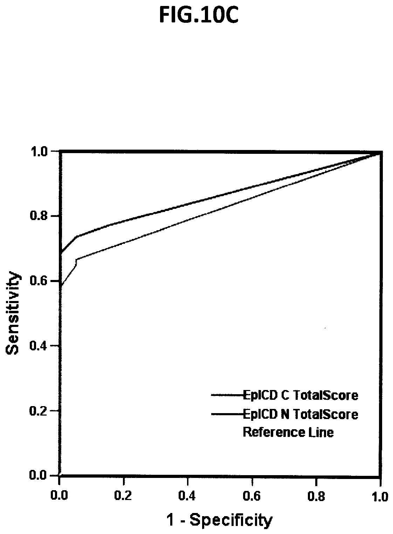

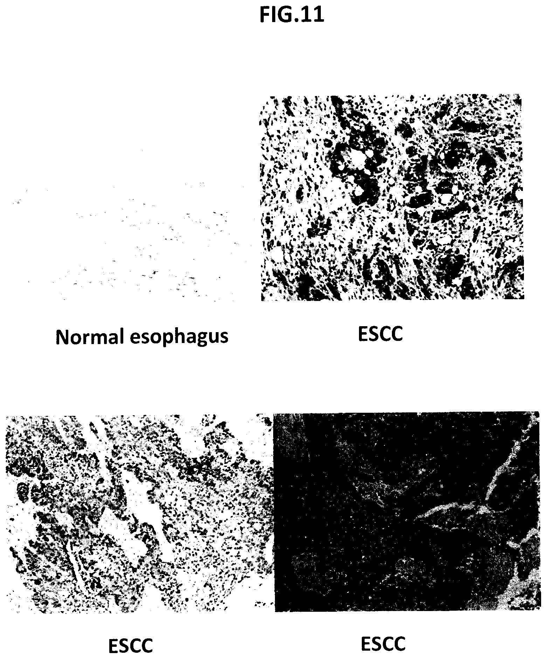

The distribution of Ep-ICD in nuclei of epithelial cells in normal and malignant breast tissues, normal and malignant prostate tissues, and in cancers of lung, colon and rectum, liver, urinary bladder, ovary and pancreas analysed are shown in the bar diagram in Figure 5. Reciever operating curves were used to determine the sensitivity and specificity of nuclear Ep-ICD in epithelial cancers. In breast cancer, nuclear Ep-ICD showed a sensitivity of 94.12% and specificity of 100%, with area under the curve of 0.968 with an IHC score cutoff value >1.7 (Figure 6). In prostate cancer, nuclear Ep-ICD showed a sensitivity of 95.56% and specificity of 100%, with area under the curve of 0.973 with an IHC score cutoff >2.67 (Figure 7). Analysis of Ep-ICD expression in head and neck normal and cancer tissues showed nuclear localization in tumor cells (Figures 8 and 9). The results of Ep-ICD, EpEx and beta-catenin expression analysis in head and neck cancer are summarized in Tables 1, 2, and 3 respectively. In head and neck cancer, nuclear Ep-ICD showed a sensitivity of 66.67% and specificity of 95%, with area under the curve of 0.822 with an IHC score cutoff value >1.7 (Figure 10). Analysis of Ep-ICD expression in esophageal cancer tissues showed nuclear localization in tumor cells (Figure11 and Table 4). The nuclear and cytoplasmic distribution of Ep-ICD in epithelial cells in normal and malignant breast tissues, normal and malignant prostate tissues, and in cancers of lung, colon and rectum, liver, urinary bladder, ovary and pancreas analysed are summarized in Table 5. Receiver operating curves analyses were carried and the sensitivity, specificity, area under the curve (AUC) values for nuclear and cytoplasmic distribution of Ep-ICD in cancers of prostate, breast, head and neck and esophagus are summarized in Table 6.

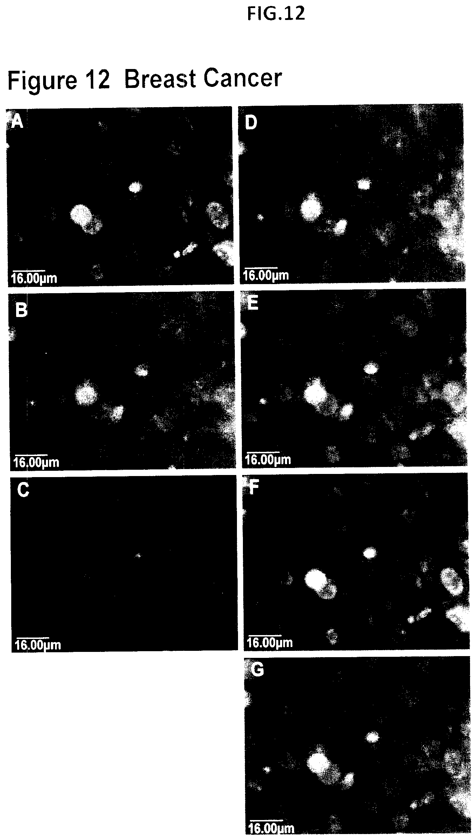

The immunohistochemical data were verified by immunofluorescence analysis. Figure 12 shows EpICD, EpEx and nuclear DNA subcellular localization in human breast carcinomas. EpICD (red), EpEx (green) and nucleic DNA (blue) were monitored with specific antibodies and DAPI, respectively. A. Nucleic DNA stained with DAPI (blue). B. Subcellular localization of EpICD (red) in breast cancer cells. C. Subcellular localization of EpEx (green) in breast cancer cells. D. Merged image of B&C showing EpICD localized in cytoplasm and nuclei. E. Merged image of A&B showing the nuclear colocalization of EpICD and DAPI. F. Merged image of A&C showing dominating DAPI nuclear staining. G. Merged image of A&B&C reshowing the nuclear colocalization of EpICD and DAPI, also the cytoplasmic EpICD.

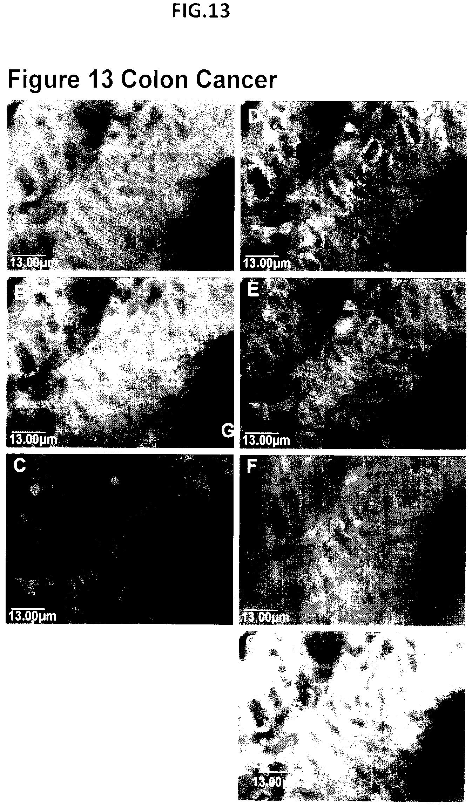

Immunofluorescence analysis of EpICD, EpEx and DNA subcellular localization in human colon carcinomas is shown in Figure 13. EpICD (red), EpEx (green) and nucleic DNA (blue) were monitored with specific antibodies and DAPI, respectively in colon cancer paraffin section. A. Nucleic DNA stained with DAPI (blue) in colon cancer cells. B. Subcellular localization of EpICD (red) in colon cancer cells. Strong cytoplasmic staining and medium level of nuclear staining of EpICD were demonstrated in colon cancer cells. C. Subcellular localization of EpEx (green) in colon cancer. Strong cytoplasmic staining and low level of membrane staining were displayed in colon cancer cells. D. Merged image of A&C showing dominating DAPI nuclear staining and strong EpEx cytoplasmic staining. E. Merged image of A&B showing the nuclear colocalization of EpICD and DAPI. F. Merged image of B&C showing EpICD colocalized with EpEx in cytoplasm and also appeared in nuclei. G. Merged image of A&B&C showing the nuclear colocalization of EpICD and nuclear DNA, also showing the cytoplasmic EpICD and EpEx in colon cancer cells.

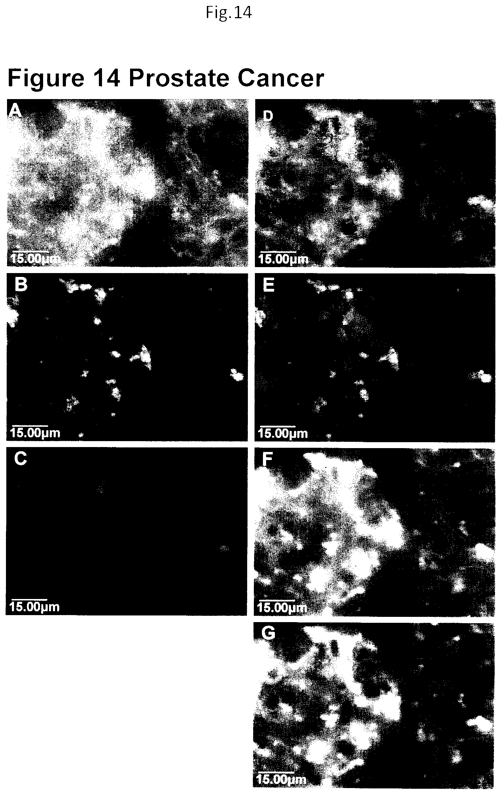

Representative immunofluorescence micrographs of EpICD, EpEx and DNA subcellular localization in human prostate carcinomas is shown in Figure 14. Subcellular localization of EpICD (red), EpEx (green) and nucleic DNA (blue) were observed with specific antibodies and DAPI, respectively in human prostate cancer paraffin section. A. Nucleic DNA stained with DAPI (blue) in prostate cancer cells. B. Subcellular localization of EpICD (red) in prostate cancer cells. Strong cytoplasmic staining and medium level of nuclear staining of EpICD were demonstrated in prostate cancer cells. C. Subcellular localization of EpEx (green) in prostate cancer cells. Strong cytoplasmic staining and low level of membrane staining were observed. D. Merged image of A&C showing DAPI nuclear DNA staining, weak EpEx membrane staining and strong cytoplasmic staining in prostate cancer. E. Merged image of A&B showing the nuclear colocalization of EpICD and DAPI. F. Merged image of B&C showing EpICD colocalized with EpEx in cytoplasm. G. Merged image of A&B&C showing the nuclear colocalization of EpICD and nuclear DNA, also showing the cytoplasmic EpICD and EpEx in prostate cancer cells.

**Table 1. ANALYSIS OF EpICD PROTEIN EXPRESSION IN HEAD AND NECK CANCER**

| **Clinico-pathological Features** | **Total Cases** | **Cytoplasmic N** | **Positivity (%)** | **p-value** | **OR (95% CI)** | **Nuclear N** | **Positivity (%)** | **p-value** | **OR (95% CI)** |
|---|---|---|---|---|---|---|---|---|---|
| Normal | 20 | 3 | 15.0 | | | 2 | 10 | | |
| HNSCC | 57 | 44 | 77.2 | **<0.00 1** | 28.77 (5.86-141.1 3) | 39 | 68.4 | **<0.00 1** | 39.0(4 .82-315.2 1) |

**Table 2. ANALYSIS OF EpEx PROTEIN EXPRESSION IN HEAD AND NECK CANCER**

| **Clinicopathological Features** | **Total Cases** | **Cytoplasmic N** | **Positivity (%)** |
|---|---|---|---|
| Normal | 20 | 0 | |
| HNSCC | 57 | 5 | 8.7 |

**Table 3. ANALYSIS OF B-Cat PROTEIN EXPRESSION IN HEAD AND NECK CANCER**

| **Clinicopathological Features** | **Total Cases** | **Membranous N** | **Positivity (%)** | **Cytoplasmic N** | **Positivity (%)** |
|---|---|---|---|---|---|
| Normal | 20 | 1 | 5.0 | 0 | |
| HNSCC | 57 | 3 | 5.2 | 4 | 7.0 |

**Table 4. ANALYSIS OF EpICD PROTEIN EXPRESSION IN ESOPHAGEAL CANCER**

| **Clinicopathological Features** | **Total Cases** | **Only Cytoplasmic N%** | **Nuclear N(%)** | **Overall positivity N(%)** |
|---|---|---|---|---|
| Normal | 20 | 5(25) | 3(15) | 8(40) |
| ESCC | 46 | 16(35) | 16(35) | 32(70) |

**Table 5. Immunohistochemical Analysis of Ep-ICD in Normal and Cancerous Epithelia**

| **Tissue Type** | **Cancer or Normal** | **Number Tissues (n)** | **Nuclear Positive (n)** | **Nuclear Positivity (%)** | **Cytoplasmic Positive (n)** | **Cytoplasmic Positivity (%)** |
|---|---|---|---|---|---|---|
| **Prostate** | Cancer | 49 | 40 | 82 | 40 | 82 |
| | Normal | 9 | 2 | 22 | 1 | 11 |
| | BPH | 21 | 0 | 0 | 1 | 5 |
| **Breast** | Cancer | 38 | 31 | 82 | 32 | 84 |
| | Normal | 25 | 0 | 0 | 0 | 0 |
| **Lung** | Cancer | 59 | 47 | 80 | 56 | 95 |
| **Colon** | Cancer | 59 | 49 | 83 | 46 | 78 |
| **Ovarian** | Cancer | 10 | 10 | 100 | 10 | 100 |
| **Pancreas** | Cancer | 10 | 3 | 30 | 2 | 20 |
| **Liver** | Cancer | 9 | 9 | 100 | 8 | 89 |
| **Bladder** | Cancer | 10 | 9 | 90 | 9 | 90 |

| | | | | | | |
|---|---|---|---|---|---|---|
| Note: A cutoff value of 4 was used to determine positivity. BPH, benign prostate hyperplasia. | | | | | | |

**Table 6. Biomarker Analysis of Nuclear and Cytoplasmic Ep-ICD Expression in Epithelial Cancers**

| **Ep-ICD Nuclear Staining Scores** | **AUC** | **Sensitivity (%)** | **Specificity (%)** | **PPV (%)** | **NPV (%)** | **Asymptotic Sig.** |
|---|---|---|---|---|---|---|
| Prostate Cancer vs. Normal | 0.867 | 82 | 78 | 95 | 44 | 0.001 |
| Breast Cancer vs. Normal | 0.905 | 82 | 100 | 100 | 78 | 0.000 |
| HNSCC vs. Normal | 0.822 | 65 | 95 | 97 | 49 | 0.000 |
| ESCC vs. Normal | 0.630 | 37 | 90 | 90 | 38 | 0.001 |

| **Ep-ICD Cytoplasmic Staining Scores** | **AUC** | **Sensitivity (%)** | **Specificity (%)** | **PPV (%)** | **NPV (%)** | **Asymptotic Sig.** |
|---|---|---|---|---|---|---|
| Prostate Cancer vs. Normal | 0.880 | 82 | 89 | 98 | 47 | 0.000 |
| Breast Cancer vs. Normal | 0.928 | 84 | 100 | 100 | 81 | 0.000 |
| HNSCC vs. Normal | 0.864 | 74 | 95 | 98 | 56 | 0.000 |
| ESCC vs. Normal | 0.758 | 70 | 70 | 84 | 50 | 0.001 |

| | | | | | | |
|---|---|---|---|---|---|---|
| Note: A cutoff value of 4 was used to determine positivity. | | | | | | |

### Sequence Listing

SEQ ID NO. 1
   NP_002345 
MAPPQVLAFG LLLAAATATF AAAQEECVCE NYKLAVNCFV NNNRQCQCTS VGAQNTVICS
60
KLAAKCLVMK AEMNGSKLGR RAKPEGALQN NDGLYDPDCD ESGLFKAKQC NGTSTCWCVN
120
TAGVRRTDKD TEITCSERVR TYWIIIELKH KAREKPYDSK SLRTALQKEI TTRYQLDPKF
180
ITSILYENNV ITIDLVQNSS QKTQNDVDIA DVAYYFEKDV KGESLFHSKK MDLTVNGEQL
240
DLDPGQTLIY YVDEKAPEFS MQGLKAGVIA VIVVVVIAVV AGIVVLVISR KKRMAKYEKA
300
EIKEMGEMHR ELNA
314
SEQ ID NO. 2
   UniProtKB/TrEMBL Q6FG26 (HUMAN) 
atggcgcccc cgcaggtcct cgcgttcggg cttctgcttg ccgcggcgac ggcgactttt
60
gccgcagctc aggaagaatg tgtctgtgaa aactacaagc tggccgtaaa ctgctttgtg
120
aataataatc gtcaatgcca gtgtacttca gttggtgcac aaaatactgt catttgctca
180
aagctggctg ccaaatgttt ggtgatgaag gcagaaatga atggctcaaa acttgggaga
240
agagcaaaac ctgaaggggc cctccagaac aatgatgggc tttatgatcc tgactgcgat
300
gagagcgggc tctttaaggc caagcagtgc aacggcacct ccatgtgctg gtgtgtgaac
360
actgctgggg tcagaagaac agacaaggac actgaaataa cctgctctga gcgagtgaga
420
acctactgga tcatcattga actaaaacac aaagcaagag aaaaacctta tgatagtaaa
480
agtttgcgga ctgcacttca gaaggagatc acaacgcgtt atcaactgga tccaaaattt
540
atcacgagta ttttgtatga gaataatgtt atcactattg atctggttca aaattcttct
600
caaaaaactc agaatgatgt ggacatagct gatgtggctt attattttga aaaagatgtt
660
aaaggtgaat ccttgtttca ttctaagaaa atggacctga cagtaaatgg ggaacaactg
720
gatctggatc ctggtcaaac tttaatttat tatgttgatg aaaaagcacc tgaattctca
780
atgcaggtct aaaagctgg tgttattgct gttattgtgg ttgtggtgat agcagttgtt
840
gctggaattg ttgtgctggt tatttccaga aagaagagaa tggcaaagta tgagaaggct
900
gagataaagg agatgggtga gatgcatagg gaactcaatg ca
942
SEQ ID NO. 3 
1 CGGCGAGCGA GCACCTTCGA CGCGGTCCGG GGACCCCCTC GTCGCTGTCC TCCCGACGCG
61 GACCCGCGTG CCCCAGGCCT CGCGCTGCCC GGCCGGCTCC TCGTGTCCCA CTCCCGGCGC
121 ACGCCCTCCC GCGAGTCCCG GGCCCCTCCC GCGCCCCTCT TCTCGGCGCG CGCGCAGCAT
181 GGCGCCCCCG CAGGTCCTCG CGTTCGGGCT TCTGCTTGCC GCGGCGACGG CGACTTTTGC FORWARD PRIMER
241 CGCAGCTCAG GAAGAATGTG TCTGTGAAAA CTACAAGCTG GCCGTAAACT GCTTTGTGAA SiRNA TARGET 1
301 TAATAATCGT CAATGCCAGT GTACTTCAGT TGGTGCACAA AATACTGTCA TTTGCTCAAA
361 GCTGGCTGCC AAATGTTTGG TGATGAAGGC AGAAATGAAT GGCTCAAAAC TTGGGAGAAG
421 AGCAAAACCT GAAGGGGCCC TCCAGAACAA TGATGGGCTT TATGATCCTG ACTGCGATGA
481 GAGCGGGCTC TTTAAGGCCA AGCAGTGCAA CGGCACCTCC ACGTGCTGGT GTGTGAACAC
541 TGCTGGGGTC AGAAGAACAG ACAAGGACAC TGAAATAACC TGCTCTGAGC GAGTGAGAAC
601 CTACTGGATC ATCATTGAAC TAAAACACAA AGCAAGAGAA AAACCTTATG ATAGTAAAAG
661 TTTGCGGACT GCACTTCAGA AGGAGATCAC AACGCGTTAT CAACTGGATC CAAAATTTAT SiRNA TARGET 2
721 CACGAGTATT TTGTATGAGA ATAATGTTAT CACTATTGAT CTGGTTCAAA ATTCTTCTCA
781 AAAAACTCAG AATGATGTGG ACATAGCTGA TGTGGCTTAT TATTTTGAAA AAGATGTTAA
841 AGGTGAATCC TTGTTTCATT CTAAGAAAAT GGACCTGACA GTAAATGGGG AACAACTGGA
901 TCTGGATCCT GGTCAAACTT TAATTTATTA TGTTGATGAA AAAGCACCTG AATTCTCAAT
961 GCAGGGTCTA AAAGCTGGTG TTATTGCTGT TATTGTGGTT GTGGTGATAG CAGTTGTTGC
1021 TGGAATTGTT GTGCTGGTTA TTTCCAGAAA GAAGAGAATG GCAAAGTATG AGAAGGCTGA
1081 GATAAAGGAG ATGGGTGAGA TGCATAGGGA ACTCAATGCA TAACTATATA ATTTGAAGAT SiRNA TARGET 3
1141 TATAGAAGAA GGGAAATAGC AAATGGACAC AAATTACAAA TGTGTGTGCG TGGGACGAAG
1201 ACATCTTTGA AGGTCATGAG TTTGTTAGTT TAACATCATA TATTTGTAAT AGTGAAACCT
1261 GTACTCAAAA TATAAGCAGC TTGAAACTGG CTTTACCAAT CTTGAAATTT GACCACAAGT
1321 GTCTTATATA TGCAGATCTA ATGTAAATCC CAGAACTTGG ACTCCATCGT TAAAATTATT SiRNA TARGET 4
1381 TATGTGTAAC ATTCAAATGT GTGCATTAAA TATGCTTCCA CAGTAAAATC TGAAAAACTG
1441 ATTTGTGATT GAAAGCTGCC TTTCTATTTA CTTGAGTCTT GTACATACAT ACTTTTTTAT
1501 GAGCTATGAA ATAAAACATT TTAAACTG:
SEQ ID NO. 4
   NM_002354_11
   1 tccagaaaga agagaatggc aaagtatgag aaggctgaga taaaggagat gggtgagatg
   61 catagggaac tcaatgca
SEQ ID NO. 5
   AACTACAAGCTGGCCGTAAAC
SEQ ID NO. 6
   GCTGGTGTGTGAACACTGCT
SEQ ID NO. 7
   AGAAGGAGATCACAACGCGTTATCAACTGGATCC
SEQ ID NO. 8
   AACGCGTTATCAACTGGATCC
SEQ ID NO. 9
   AAGGAGATGGGTGAGATGCA
SEQ ID NO. 10
   AATCCAGAACTTGGACTCCAT

## Claims

1. A method for diagnosing an epithelial cancer in a subject comprising:
(a) detecting a level of an EpICD polypeptide or a polynucleotide encoding an EpICD polypeptide in a sample from the subject, wherein the EpICD polypeptide consists of a 26 amino acid cytoplasmic domain of EpCAM; and
(b) comparing the level detected in the subject's sample to levels detected for a predetermined standard, wherein an increased level of EpICD polypeptide or polynucleotide encoding an EpICD polypeptide in the sample from the subject over that of the control level is
indicative of epithelial cancer, wherein the epithelial cancer is breast cancer, prostate cancer, lung cancer, pancreatic cancer, urinary bladder cancer, ovarian cancer, liver cancer, head and neck cancer or esophageal cancer.

2. A method of claim 1 wherein the predetermined standard is a control level obtained from samples of the same type from subjects who do not have the epithelial cancer.

3. A method of claim 1 or 2, further comprising:
(c) detecting in the sample a level of an EpEX polypeptide or a polynucleotide encoding an EpEx polypeptide; and
(d) comparing the EpEx level detected in the subject's sample to levels detected for a second predetermined standard, wherein a decreased level of EpEx polypeptide or polynucleotide
encoding an EpEx polypeptide in the sample from the subject over that of the second control level is further indicative of the epithelial cancer.

4. A method of claim 3, wherein the second predetermined standard is a second control level obtained from samples of the same type from subjects who do not have the epithelial cancer.

5. A method of any of the preceding claims, wherein detection of EpICD or EpEx is obtained using at least one reagent capable of measuring levels of EpICD or EpEX polypeptides or polynucleotides encoding EpICD or EpEx polypeptides.

6. A method for diagnosing an increased risk of an epithelial cancer, in a subject,
the method comprising a) contacting a first sample from a subject at a first time with a diagnostic reagent that measures a first level of an EpICD polypeptide, wherein the EpICD polypeptide consists of a 26 amino acid cytoplasmic domain of EpCAM; and b) diagnosing an increased risk of an epithelial cancer wherein the epithelial cancer is breast cancer, prostate cancer, lung cancer, pancreatic cancer, urinary bladder cancer, ovarian cancer, liver cancer, head and neck cancer or esophageal cancer in the subject based upon an increased level of EpICD polypeptide in the sample from the subject over that of (i) a first control level of EpICD polypeptide obtained from samples of the same type taken from subjects who do not have the epithelial cancer; or (ii) an earlier sample level of EpICD polypeptide obtained from samples of the same type taken from the same subject at an earlier time.

7. A method of any preceding claim, wherein levels of EpICD polypeptide are measured by detecting, directly or indirectly, the interaction of the EpICD polypeptide with an antibody specific for the EpICD polypeptide.

8. A method of claim 7, wherein the antibody is labeled with an enzyme, fluorescent, luminescent or radioactive material.

9. A method of claim 8, further comprising performing a step selected from the group consisting of a counter immuno-electrophoresis, a radioimmunoassay, radioimmunoprecipitation assay, an enzyme-linked immunosorbent assay, a dot blot assay or an inhibition of competition assay and a sandwich assay using said antibody.

10. A method of claim 6, wherein the subject does not have an increased risk of developing an epithelial cancer if the first level is the same as either the first control level or the earlier sample level.

11. A method for monitoring the progression of an epithelial cancer in a subject, the method comprising: (a) detecting an EpICD polypeptide or polynucleotide encoding an EpICD polypeptide in a sample from a patient at a first time point, wherein the EpICD polypeptide consists of a 26 amino acid cytoplasmic domain of EpCAM; (b) repeating step (a) at a subsequent point in time; and (c) comparing levels detected in steps (a) and (b), and thereby monitoring the progression of the cancer, wherein the epithelial cancer is breast cancer, prostate cancer, lung cancer, pancreatic cancer, urinary bladder cancer, ovarian cancer, liver cancer, head and neck cancer or esophageal cancer.

12. A method of any preceding claim wherein the EpICD Polypeptide consists of amino acids 289 to 314 of SEQ ID NO. 1.

13. A method of any preceding claim wherein the sample is breast, prostate, lung, pancreatic, bladder, ovarian, head, neck, esophageal or liver tissue.

14. A method of any preceding claim wherein the method shows a sensitivity and/or specificity of greater than 90%.

15. Use of a kit in a method according to any of claims 1 to 14, comprising agents that hybridize to or amplify polynucleotides encoding EpICD polypeptides, wherein the EpICD polypeptide consists of a 26 amino acid cytoplasmic domain of EpCAM.

## Patentansprüche

1. Verfahren zum Diagnostizieren eines epithelialen Krebses in einem Subjekt, umfassend:
(a) Bestimmen eines Levels von einem EpICD-Polypeptid oder einem Polynukleotid, das ein EpICD-Polypeptid kodiert, in einer Probe von dem Subjekt, wobei das EpICD-Polypeptid aus einer 26 Aminosäuren langen zytoplasmatischen Domäne von EpCAM besteht; und
(b) Vergleichen des Levels, das in der Probe des Subjektes bestimmt wurde, mit Levels, die für einen vorher bestimmten Standard bestimmt wurden, wobei ein erhöhtes Level von EpICD-Polypeptid oder Polynukleotid, das ein EpICD-Polypeptid kodiert, in der Probe von dem Subjekt, das über dem Kontrolllevel liegt, auf epithelialen Krebs hinweist, wobei der epitheliale Krebs Brustkrebs, Prostatakrebs, Lungenkrebs, Bauchspeicheldrüsenkrebs, Harnblasenkrebs, Eierstockkrebs, Leberkrebs, Kopf- und Halskrebs oder Speiseröhrenkrebs ist.

2. Verfahren nach Anspruch 1, wobei der vorher bestimmte Standard ein Kontrolllevel ist, der aus Proben des gleichen Typs von Subjekten gewonnen wurde, die keinen epithelialen Krebs haben.

3. Verfahren nach Anspruch 1 oder 2, ferner umfassend:
(c) Bestimmen eines Levels eines EpEX-Polypeptids oder eines Polynukleotids, das ein EpEx-Polypeptid kodiert, in der Probe; und
(d) Vergleichen des EpEx-Levels, das in der Probe des Subjektes bestimmt wurde, mit Levels die für einen zweiten vorher bestimmten Standard bestimmt wurden, wobei ein verringertes Level von EpEx-Polypeptid oder Polynukleotid, das ein EpEx-Polypeptid kodiert, in der Probe von dem Subjekt, der über dem des zweiten Kontrolllevel liegt, ferner auf den epithelialen Krebs hinweist.

4. Verfahren nach Anspruch 3, wobei der zweite vorher bestimmte Standard ein zweiter Kontrolllevel ist, der aus Proben des gleichen Typs von Subjekten erhalten wurde, die keinen epithelialen Krebs haben.

5. Verfahren nach einem der vorhergehenden Ansprüche, wobei der Nachweis von EpICD oder EpEx durch die Verwendung mindestens eines Reagenz erhalten wird, das fähig ist, Levels von EpICD- oder EpEx-Polypeptiden oder Polynukleotiden, die EpICD- oder EpEx-Polypeptide kodieren, zu messen.

6. Verfahren zum Diagnostizieren eines erhöhten Risikos eines epithelialen Krebses bei einem Subjekt, wobei das Verfahren umfasst a) Inkontaktbringen einer ersten Probe von einem Subjekt zu einem ersten Zeitpunkt mit einem diagnostischen Reagenz, das ein erstes Level eines EpICD-Polypeptids misst, wobei das EpICD-Polypeptid aus einer 26 Aminosäuren langen zytoplasmatischen Domäne des EpCAM besteht; und b) Diagnostizieren eines erhöhten Risikos eines epithelialen Krebses, wobei der epitheliale Krebs Brustkrebs, Prostatakrebs, Lungenkrebs, Bauchspeicheldrüsenkrebs, Harnblasenkrebs, Eierstockkrebs, Leberkrebs, Kopf- und Halskrebs oder Speiseröhrenkrebs bei dem Subjekt ist, auf der Grundlage eines erhöhten EpICD-Polypeptid-Levels in der Probe von dem Subjekt, das über dem von (i) einem ersten Kontrolllevel von EpICD-Polypeptid aus Proben des gleichen Typs liegt, die von Subjekten erhalten wurden, die keinen epithelialen Krebs haben; oder (ii) einem früheren Probenlevel von EpICD-Polypeptid, das aus Proben des gleichen Typs erhalten wurde, die von demselben Subjekt zu einem früheren Zeitpunkt entnommen wurden, liegt.

7. Verfahren nach einem der vorhergehenden Ansprüche, wobei die Levels von EpICD-Polypeptid durch direktes oder indirektes Bestimmen der Interaktion des EpICD-Polypeptids mit einem Antikörper, der für EpICD-Polypeptide spezifisch ist, gemessen werden.

8. Verfahren nach Anspruch 7, wobei der Antikörper mit einem Enzym, fluoreszierendem, lumineszierendem oder radioaktivem Material markiert ist.

9. Verfahren nach Anspruch 8, ferner umfassend Durchführen eines Schritts, der aus der Gruppe ausgewählt wird, die aus einer Immuno-Gegenstromelektrophorese, einem Radioimmunoassay, Radioimmunopräzipitationsassay, einem Enzym-gekoppelten Immunosorptionsassay, einem Dot-Blot-Assay oder einem kompetitiven Inhibitionsassays und einem Sandwich-Assay unter Verwendung des Antikörpers besteht.

10. Verfahren nach Anspruch 6, wobei das Subjekt kein erhöhtes Risiko für das Entwickeln von epithelialem Krebs hat, wenn das erste Level entweder dem ersten Kontrolllevel oder dem früheren Probenlevel gleicht.

11. Verfahren zum Überwachen des Fortschreitens eines epithelialen Krebses in einem Subjekt, wobei das Verfahren umfasst: (a) Bestimmen eines EpICD-Polypeptids oder Polynukleotids, das ein EpICD-Polypeptid kodiert, in einer Probe von einem Subjekt zu einem ersten Zeitpunkt, wobei das EpICD-Polypeptid aus einer 26 Aminosäuren langen zytoplasmatischen Domäne von EpCAM besteht; (b) Wiederholen von Schritt (a) zu einem späteren Zeitpunkt; und (c) Vergleichen von Levels, die in den Schritten (a) und (b) bestimmt wurden, und wobei das Fortschreiten des Krebses überwacht wird, wobei der epitheliale Krebs Brustkrebs, Prostatakrebs, Lungenkrebs, Bauchspeicheldrüsenkrebs, Harnblasenkrebs, Eierstockkrebs, Leberkrebs, Kopf- und Halskrebs oder Speiseröhrenkrebs ist.

12. Verfahren nach einem der vorhergehenden Ansprüche, wobei das EpICD-Polypeptid aus den Aminosäuren 289 bis 314 der SEQ ID NR. 1 besteht.

13. Verfahren nach einem der vorhergehenden Ansprüche, wobei die Probe Brust-, Prostata-, Lungen-, Bauchspeicheldrüsen-, Blasen-, Eierstock-, Kopf-, Hals-, Speiseröhren- oder Lebergewebe ist.

14. Verfahren nach einem der vorhergehenden Ansprüche, wobei das Verfahren eine Sensibilität und/oder Spezifität von über 90 % zeigt.

15. Verwendung eines Kits in einem Verfahren nach einem der Ansprüche 1 bis 14, das Agenzien umfasst, die an Polynukleotide hybridisieren oder diese vervielfachen, die EpICD-Polypeptide kodieren, wobei das EpICD-Polypeptid aus einer 26 Aminosäuren langen zytoplasmatischen Domäne von EpCAM besteht.

## Revendications

1. Un procédé de diagnostic d'un cancer épithélial chez un sujet, comprenant les étapes consistant à :
(a) détecter un niveau d'un polypeptide EpICD ou d'un polynucléotide codant pour un polypeptide EpICD dans un échantillon provenant du sujet, dans lequel le polypeptide EpICD est constitué d'un domaine cytoplasmique d'EpCAM de 26 acides aminés ; et
(b) comparer le niveau détecté dans l'échantillon du sujet à des niveaux détectés pour une norme prédéterminée, dans lequel un niveau augmenté de polypeptide EpICD ou de polynucléotide codant un polypeptide EpICD dans l'échantillon provenant du sujet par rapport au niveau témoin est indicateur d'un cancer épithélial, dans lequel le cancer épithélial est le cancer du sein, le cancer de la prostate, le cancer du poumon, le cancer du pancréas, le cancer de la vessie, le cancer de l'ovaire, le cancer du foie, le cancer des voies aérodigestives supérieures ou le cancer de l'oesophage.

2. Un procédé selon la revendication 1, dans lequel la norme prédéterminée est un niveau témoin obtenu à partir d'échantillons du même type provenant de sujets qui n'ont pas de cancer épithélial.

3. Un procédé selon la revendication 1 ou 2, comprenant en outre les étapes consistant à :
(c) détecter dans l'échantillon un niveau d'un polypeptide EpEx ou d'un polynucléotide codant pour un polypeptide EpEx ; et
(d) comparer le niveau EpEx détecté dans l'échantillon du sujet avec des niveaux détectés pour une deuxième norme prédéterminée, dans lequel un niveau réduit de polypeptide EpEx ou de polynucléotide codant un polypeptide EpEx dans l'échantillon provenant du sujet par rapport à celui du deuxième niveau témoin est également indicateur du cancer épithélial.

4. Un procédé selon la revendication 3, dans lequel la deuxième norme prédéterminée est un deuxième niveau témoin obtenu à partir d'échantillons du même type provenant de sujets qui n'ont pas de cancer épithélial.

5. Un procédé selon l'une quelconque des revendications précédentes, dans lequel la détection d'EpICD ou d'EpEx est obtenue en utilisant au moins un réactif capable de mesurer des niveaux de polypeptides EpICD ou EpEx ou de polynucléotides codant pour des polypeptides EpICD ou EpEx.

6. Un procédé de diagnostic d'un risque accru d'un cancer épithélial, chez un sujet, le procédé comprenant les étapes consistant à a) mettre en contact un premier échantillon provenant d'un sujet à un premier instant avec un réactif de diagnostic qui mesure un premier niveau d'un polypeptide EpICD, dans lequel le polypeptide EpICD est constitué d'un domaine cytoplasmique d'EpCAM de 26 acides aminés ; et b) diagnostiquer un risque accru d'un cancer épithélial, dans lequel le cancer épithélial est le cancer du sein, le cancer de la prostate, le cancer du poumon, le cancer du pancréas, le cancer de la vessie, le cancer de l'ovaire, le cancer du foie, le cancer des voies aérodigestives supérieures ou le cancer de l'oesophage chez le sujet, sur la base d'un niveau accru de polypeptide EpICD dans l'échantillon provenant du sujet par rapport à (i) un premier niveau témoin de polypeptide EpICD obtenu à partir d'échantillons du même type provenant de sujets qui n'ont pas de cancer épithélial ; ou (ii) un niveau d'un échantillon antérieur de polypeptide EpICD obtenu à partir d'échantillons du même type provenant du même sujet à un moment antérieur.

7. Un procédé selon l'une quelconque des revendications précédentes, dans lequel les niveaux de polypeptide EpICD sont mesurés en détectant, directement ou indirectement, l'interaction du polypeptide EpICD avec un anticorps spécifique du polypeptide EpICD.

8. Un procédé selon la revendication 7, dans lequel l'anticorps est marqué avec une enzyme, un matériau fluorescent, luminescent ou radioactif.

9. Un procédé selon la revendication 8, comprenant en outre une étape choisie dans le groupe constitué d'un comptage par immuno-électrophorèse, un dosage radio-immunologique, un dosage par radioimmunoprécipitation, un dosage immunoenzymatique, un dosage dot blot ou un dosage par inhibiteur compétitif et un dosage en sandwich utilisant ledit anticorps.

10. Un procédé selon la revendication 6, dans lequel le sujet n'a pas un risque accru de développer un cancer épithélial si le premier niveau est le même que le premier niveau témoin ou le niveau de l'échantillon antérieur.

11. Un procédé de surveillance de la progression d'un cancer épithélial chez un sujet, le procédé comprenant les étapes consistant à : (a) détecter un polypeptide EpICD ou un polynucléotide codant un polypeptide EpICD dans un échantillon provenant d'un patient à un premier instant, dans lequel le polypeptide EpICD est constitué d'un domaine cytoplasmique d'EpCAM de 26 acides aminés ; (b) répéter l'étape (a) à un instant postérieur ; et (c) comparer les niveaux détectés aux étapes (a) et (b), et surveiller ainsi la progression du cancer, dans lequel le cancer épithélial est le cancer du sein, le cancer de la prostate, le cancer du poumon, le cancer du pancréas, le cancer de la vessie, le cancer de l'ovaire, le cancer du foie, le cancer des voies aérodigestives supérieures ou le cancer de l'oesophage.

12. Un procédé selon l'une quelconque des revendications précédentes, dans lequel le polypeptide EpICD est constitué d'acides aminés 289 à 314 de SEQ ID No 1.

13. Un procédé selon l'une quelconque des revendications précédentes, dans lequel l'échantillon est du tissu mammaire, du tissu de la prostate, du poumon, du pancréas, de la vessie, des ovaires, des voies aérodigestives supérieures, de l'oesophage ou du foie.

14. Un procédé selon l'une quelconque des revendications précédentes, dans lequel le procédé présente une sensibilité et/ou spécificité supérieure à 90 %.

15. L'utilisation d'un kit dans un procédé selon l'une quelconque des revendications 1 à 14, comprenant des agents qui s'hybrident avec ou amplifient des polynucléotides codant des polypeptides EpICD, dans laquelle le polypeptide EpICD est constitué d'un domaine cytoplasmique d'EpCAM de 26 acides aminés.
